Europäisches Patentamt

European Patent Office

Office européen des brevets

(11) Publication number: **0 554 064 A1**

# EUROPEAN PATENT APPLICATION

(21) Application number: **93300573.8**

(22) Date of filing: **27.01.93**

(51) Int. Cl.5: **A61K 39/00, C12N 15/62,**
**C07K 15/08, C07K 13/00,**
**C07H 21/04, A61K 39/395,**
**C12N 15/63, G01N 33/566,**
**G01N 33/68**

The applicant has subsequently filed a sequence listing and declared, that it includes no new matter.

(30) Priority: **28.01.92 IL 100783**

(43) Date of publication of application:
**04.08.93 Bulletin 93/31**

(84) Designated Contracting States:
**CH DE ES FR GB IT LI SE**

(71) Applicant: **YEDA RESEARCH AND**
**DEVELOPMENT CO. LTD.**
**P.O. Box 95**
**Rehovot 76100(IL)**

(72) Inventor: **Schechter, Israel**
**Beit Brazil 1, Weizmann Institute of Science**
**Rehovot(IL)**
Inventor: **Markovics, Alexander**
**P.O. Box 417**
**Kochav Yair(IL)**
Inventor: **Ram, Daniela**
**8 Hakalanit Street**
**Carmei Yosef 72910(IL)**

(74) Representative: **Collier, Jeremy Austin Grey**
**J.A.Kemp & Co., 14 South Square, Gray's Inn**
**London WC1R 5LX (GB)**

(54) **Vaccine against schistosomiasis.**

(57) An immunogenically and antigenically active protein capable of eliciting immunity against *Schistosoma* parasites is provided, as are vaccines containing the protein, that are capable of eliciting in humans or animals an immune response that affords protection against infection by *Schistosoma* parasites. Antibodies raised against the protein, DNA molecules encoding the protein, vectors containing the DNA molecules, host cells transformed by the vectors, and diagnostic methods using the antibodies or the proteins are also provided.

FIG. 8

## FIELD OF THE INVENTION

The present invention concerns surface antigens of *Schistosoma mansoni* which causes Bilharzia in humans and animals, which antigens may be useful for preparing vaccines against this disease.

More specifically, the present invention concerns a *S. mansoni* integral membrane protein of molecular weight of about 25 KD (hereinafter "SmIMP25") which is a surface antigen of *S. mansoni*, which is useful for preparing a vaccine against Bilharzia.

Further, the present invention also concerns immunogenic fragments of SmIMP25 which are also useful for preparing vaccines against Bilharzia.

In addition, the present invention concerns recombinant DNA methods for the preparation of SmIMP25 and its immunogenic fragments.

## BACKGROUND OF THE INVENTION AND PRIOR ART

In the following description reference will be made to a number of publications, the full details of which are to be found in the section entitled **"References"** at the end of the Examples.

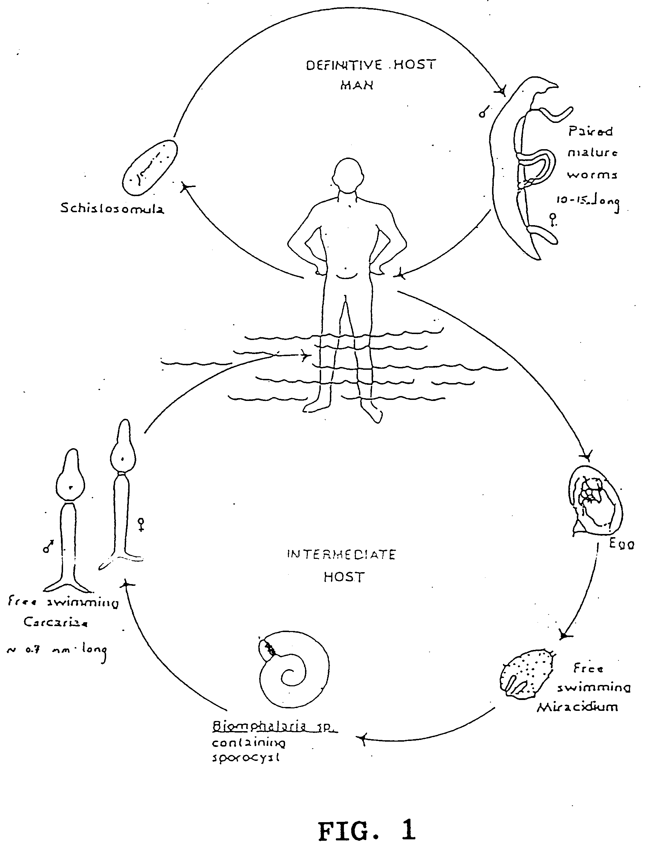

*Schistosomes* are helminth parasites of the class *Trematoda* that cause Bilharzia in man and animals. They have a complex life cycle (see Fig. 1) involving parasitizing molluscan and vertebrate hosts, and two short periods of larvae living freely in water. The adult female and male worms are paired and reside in the veins of the portal system or urinary bladder of vertebrate hosts. The females lay eggs which are excreted in the host's faeces or urine. On contact with fresh water the eggs hatch to liberate miracidia that swim in water, penetrate a suitable snail, and migrate to the hepatopancreas where they transform to sporocysts. The sporocysts, by asexual division, give rise to many second generation sporocysts that differentiate into an infective stage, the cercaria. The cercariae penetrate through the intact skin of the final host, loose their tails, and rapidly undergo profound changes in the structure and physiological properties of the surface layer (the tegument) to become schistosomula adapted to parasitic life in the vertebrate host. The schistosomula migrate to the lung and then to the liver, where they grow and differentiate into mature worms. The complete cycle, from egg release to full sexual maturity takes 2.5-3 months.

Bilharzia (schistosomiasis) is a chronic debilitating disease which creates major socio-economic and health problems in large portions of Africa, Asia and South America. Human schistosomiasis is caused mainly by *S.mansoni* (Africa, South America), *S. haematobium* (Africa) and *S. japonicum* (Far East), and occasionally by *S. mecongi* (South-Eastern Asia) and *S. intercalatum* (Africa). It is estimated that 250 million people are infected, 500 million are exposed to threat of infection and the disease is responsible for about 800,000 deaths per year. *S. bovis* (Mediterranean, North and Central Africa), *S. matteei* (South Africa) and *S. japonicum* (Far East) are infective to cattle and sheep, causing severe damage to livestock particularly in countries where undernourishment is a major problem.

The control of the disease requires a multi-disciplinary approach involving public health measures, snail control, drugs and vaccine. Public health measures and snail control are mostly impractical and too expensive to implement in the third world countries due to poverty, poor sanitation, primitive agriculture practices, and the large size of endemic regions. Praziquantel is a very effective anti-schistosome drug that has replaced other moderately effective and toxic drugs. However, after a decade of widespread use of praziquantel it has become clear that the drug alone is not sufficient to control the disease. It was found that treated patients, mainly children, are readily reinfected in endemic areas because the disease does not elicit effective immunity. It should be noted that although Bilharzia patients and infected animals develop a considerable immune response against the parasite this provides only partial protection against reinfection.

It has therefore been a long-felt want to produce an effective protective vaccine. However, all attempts to date of preparing such vaccines have met with only partial success:

Cattle vaccinated with highly $\gamma$-irradiated schistosomula were found to be partially protected, from infection by such parasites. However, the limited supply of the parasites and safety problems associated with the use of live radiation-attenuated schistosomula, prohibited further development and the widespread application of such vaccines to livestock and clinical evaluation in humans.

Accordingly, attention has been directed to identify non-living immunogens which may be useful for eliciting a protective immune response against parasite infection. The surface antigens of the infective stage, cercariae and schistosomula, have been considered as major targets for protective immune responses and thus, surface antigens of both adult worm and invasive larvae, have been studied intensively as candidates for vaccine development (reviewed by Wright et al., 1991). In this respect it has been shown that active immunization by extracts of the surface tegument or by purified surface antigens, and passive immunization by the transfusion of monoclonal antibodies to a variety of surface antigens, have elicited

2

partial protection against challenge infection without cercariae(Harn et al., 1985; Tarrab-Hazdai et al., 1985; Smith and Clegg, 1985; Smithers et al., 1989). It was also found that non-surface antigens like paramyocin (Pearce et al., 1988) and the 28 KD protein encoding a subunit of glutathione-S-transferase (Taylor et al., 1988) can elicit protection against challenge infection, similar to surface antigens. These non-surface or internal antigens may be exposed to the immune apparatus of the host by the disintegration of dead parasites, secretion by the excretory system of the parasite, and/or due to rapid turnover and shedding of the surface tegument. Thus, many different antigens (located on the surface, internal or secreted) expressed in invasive larvae and adult worms, are capable of inducing partial protection (up to about 40%) against challenge infection.

However, for the development of protective vaccines in the required quantities it is essential that purified antigens become available in sufficient amounts. The above-noted antigens were prepared directly from *schistosomes* by conventional purification procedures and by immuno-affinity chromatography using monoclonal antibodies. However, since *schistosomes* do not multiply *in vitro* and growing the parasite in snails and in animals is time consuming, expensive, such methods yield only very small amounts of antigenic material.

To overcome the above obstacles recombinant DNA techniques are currently being used for large scale production of promising antigens to develop protective vaccines and diagnostic reagents and using such techniques, genes encoding potentially protective antigens have been cloned which include: paramyosin (Lanar et al., 1986); the 28 KD subunit of the glutathione-S-transferase of *S. mansoni* (Balloul et al., 1987); the 26 KD glutathione-S-transferase of *S. mansoni* (Smith et al., 1988) and *S. japonicum* (Trottein et al., 1990); several antigens of the surface tegument of 22.6 kd (Stein and David, 1986), of 25 kd (Knight et al., 1989), of 50 KD (Havercroft et al., 1988), of 18 KD (Dalton et al., 1987), and an integral membrane protein of 23 KD (Wright et al., 1990). Active immunization with recombinant paramyosin conferred 26% protection in mice (Pearce et al., 1988), and recombinant 28 KD glutathione-S-transferase conferred 67% protection in rats and 52% protection in hamsters (Balloul et al., 1987). It should be mentioned, however, that effective vaccines against schistosomiasis are not yet available for clinical use in humans (Wright et al., 1991).

Of the above antigens, those located on the surface of the parasite are considered to have greatest potential for vaccine development as these are the antigens first encountered by the host upon infection. However, concerning the above proteins it should be mentioned that:

(i) the 22.6 KD protein although present in the surface tegument of the parasite, is however, on the inside of this tegument, being undetectable on its outer surface and nothing is known yet about its immunological properties;

(ii) the 18 KD and 50 KD proteins are apparently located on the surface of schistosomula and antisera against the 50 KD protein were cytotoxic to schistosomula *in vitro*, but however, sequence data for the 18 KD and 50 KD proteins are not yet available, the nature of these proteins is still unknown and the capacity of these proteins to elicit protection against infection, i.e. their use as vaccines has not yet been evaluated; (iii) the 25 KD protein is apparently located inside the tegument of the adult worm, antibodies thereto not having reacted with the surface of schistosomula; and further, only very recently (Jeff S. et al., 1991) has the full sequence thereof been published, from which it is now known that this 25 KD protein of Knight et al. (1989) and referred to in Wright et al. (1991) is an entirely different protein from that of the present invention (see below), which also has a molecular weight (unglycosylated) of about 25 KD and is termed herein as SmIMP25; and (iv) the 23 KD protein has been sequenced, it has 218 amino acids and contains four putative transmembrane segments and is apparently located on the surface, but however, there is still no evidence concerning its capacity to elicit protection against infection.

Thus, taking all of the aforementioned into consideration it may be said that there still is no acceptable vaccine against Bilharzia in humans and that the causative parasite's antigens discovered and tested so far have failed to produce, on average, a greater than 40% protection level in animal tests, which protection level is considered a minimum before attempting to apply such potential vaccines in humans.

## OBJECTS OF THE INVENTION

It is an object of the present invention to provide an immunogenic *S−mansoni* surface antigen that is capable of eliciting an effective immune response and thus capable of protecting immunized animals and humans against infection by *Sctustosoma* parasites in general, and *S.mansoni* in particular.

It is another object of the present invention to provide immunogenic fragments of the above surface antigen or other proteins or peptides derived from said surface antigen or fragments, e.g. fusion polypeptides or analogs which substantially retain the immunogenicity and antigenicity of the whole surface antigen.

It is a further object of the present invention to provide a vaccine against Bilharzia for use in man and in livestock which comprises the above surface antigen or fragments and derivatives thereof.

It is a still further object of the present invention to provide a DNA molecule encoding the above surface antigen, fragments, or derived proteins or polypeptides which molecule may be inserted into a suitable expression vector for expression and production of the encoded protein, fragment or derived protein or polypeptide, as the case may be in suitably transformed host cells.

It is also the object of the invention to provide such above-noted host cells.

**SUMMARY OF THE INVENTION**

The present invention is based on the surprising finding that a newly discovered *Schistosoma mansoni* integral membrane protein of molecular weight of about 25 KD in the unglycosylated form (SmIMP25), fragments thereof as well as fusion polypeptide of the protein or fragments with the bacterial TrpE protein were capable, when injected into mice, of eliciting an effective immune response and affording these mice with protection of the highest level achieved by cloned antigens, against the *Schistosoma* parasite, the causal agent of the disease, Bilharzia. Sequence analysis revealed that the aforesaid SmIMP25 is distinct, i.e. a different protein entirely, from the previously described *S. mansoni* 25 KD surface antigen (see Knight et al., 1989; Wright et al., 1991; and Jeff et al., 1991 with respect to the above 25 KD antigen). Furthermore, it has been found that unlike some of the known surface antigens, the above SmIMP25 is present in all tested life stages of the parasite.

The present invention thus provides an antigenically and immunogenically active protein capable of eliciting immunity against *Schistosoma* parasites, selected from the group consisting of:

(a) *Schistosoma mansoni* integral membrane protein having in the unglycosylated form a molecular weight of about 25 KD (SmIMP25);

(b) analogs of (a) obtained by deletion, addition or replacement of one or more amino acids or obtained by glycosylation at one or more glycosylation sites of (a) without substantially affecting the immunogenicity of said protein;

(c) fragments of (a) or (b) comprising a portion of or all of the extracellular region of said protein or comprising a portion of or all of the intracellular and transmembrane region and a portion of the extracellular region of said protein, wherein said fragments substantially retain the antigenicity and immunogenicity of said protein; and

(d) fusion polypeptides comprising any one of (a), (b), or (c) fused to a suitable bacterial, phage or viral protein.

The above antigenically and immunogenically active proteins of the invention are also capable of conferring protective immunity against *Schiistosoma* parasites in humans or animals immunized with said proteins.

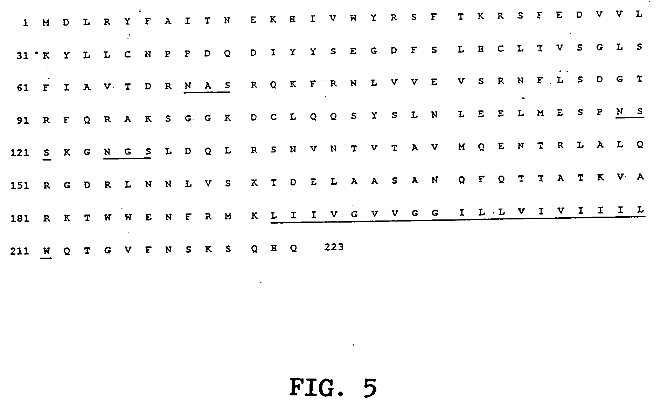

Specific embodiments of the above aspect of the invention are the following: SmIMP25 protein having the amino acid sequence depicted in Fig. 5; the analog which is designated herein as 222H which lacks the N-terminal methionine residue (residue 1 of the sequence of Fig. 5); the herein fragments designated herein as 134H and 175 having, respectively, the amino acid sequence of Fig. 5 from position 90-223 and from position 2-176, the 134H fragment comprising a part of the extracellular region and all of the transmembrane and intracellular regions of the SmIMP25 and the 175 fragment comprising only part of the extracellular region of SmIMP25; and fusion polypeptides wherein the SmIMP25, the 222H analog, the 134H and 175 fragments are fused to the TrpE protein encoded by the bacterial pATH vector, these fusion polypeptides being designated herein as SmIMP25-TrpE, 222H-TrpE, 134H-TrpE and 175-TrpE.

Further, the SmIMP25 protein and its above analogs, fragments and fusion polypeptides may be glycosylated at one or more of the N-glycosylation sites contained in the protein, analogs, fragments or fusion polypeptides, these sites being situated at positions 68-70, 119-121 and 124-126, as depicted in the sequence of Fig. 5.

The present invention also provides a vaccine comprising at least one of the proteins, analogs, fragments or fusion polypeptides of the invention and a pharmaceutically suitable excipient or carrier for administration into humans or animals, said vaccine capable of eliciting in humans or animals an anti-*Schistosome* immune response. According to one embodiment of this aspect of the invention there is provided a vaccine containing the 175-TrpE fusion polypeptide which, as is set forth hereinbelow in detail, has been shown to provide the highest level of protection both as compared to the prior art and other antigens of the invention in the mouse model of infection. According to another embodiment of this aspect of the invention there is provided a composite vaccine comprising two or more *Schistosome* antigens, at least one of which is selected from the above proteins, analogs, fragments or fusion polypeptides of the invention and at least one other antigen is selected from the group of known *Schistosoma* antigens, for example, those referred to herein above.

The present invention also provides antibodies raised against any one of the aforesaid proteins, analogs, fragments or fusion polypeptides of the invention. These antibodies can be used in, for example, diagnostic methods to detect the presence of *Schistosoma* antigens in subjects, being humans or animals, suspected of being infected by *Schistosoma* parasites or to detect the presence of said antigens in subjects infected by said parasites and undergoing treatment or following treatment against said parasites (follow-up diagnosis). With respect to this aspect of the invention it should be noted that antisera to SmIMP25, in particular, antisera to the 134H-TrpE fusion polypeptide have shown, as hereinbelow described in detail, very high levels of toxicity towards the parasite (75% killing of parasite).

The invention further provides DNA sequences encoding the aforementioned proteins, analogs, fragments and fusion products of the invention; recombinant DNA vectors containing the sequences; host cells transformed by the vectors; and recombinant DNA methods employing the transformed host cells for producing the proteins, analogs, fragments and fusion polypeptides. The above vectors may be any suitable vectors having control sequences for maintenance, propagation and expression in prokaryotic or eukaryotic host cells, and the above host cells may be any suitable prokaryotic or eukaryotic cells which can be transformed by the above vectors and in which these vectors may be suitably expressed.

Suitable vectors for use in accordance with the present invention, as noted above, are, for example bacterial, phage and viral vectors. While the bacterial and phage vectors may be any of the well known such vectors, an example of useful viral vectors are vaccinia vectors, i.e. vectors derived from vaccinia virus.

According to one embodiment of this aspect of the invention there is provided the known pATH bacterial vector into which the DNA sequences encoding SmIMP25, 222H analog and 134H and 175 fragments have been cloned in correct reading frame with the TrpE gene of the vector to obtain the SmIMP25-TrpE, 222H-TrpE, 134H-TrpE and 175-TrpE fusion polypeptides of the invention.

In another aspect, the invention provides a diagnostic method for detecting the presence of antibodies against *Schistosoma*, in subjects suspected of carrying said antibodies, using the SmIMP25 protein of the present invention or analogs, fragments or derivatives thereof according to the present invention which have essentially the same antigenicity and immunogenicity as the SmIMP25 protein.

Further aspects and advantages of the invention will be apparent from the following detailed description.

## BRIEF DESCRIPTION OF THE FIGURES

**Fig. 1** is a schematic representation of the life cycle of *S.mansoni* according to Example 1;

**Fig. 2** is a schematic representation of the SmIMP25 cDNA clones according to Example 3;

**Fig. 3** is a schematic representation of the restriction map of the 63HT cDNA clone according to Example 3;

**Fig. 4** is the nucleotide and predicted amino acid sequence of the SmIMP25 cDNA according to Examples 3 and 5;

**Fig. 5** is the predicted amino acid sequence of the SmIMP25 protein according to Example 5;

**Fig. 6** is a representation of an autoradiogram of a Northern blot to detect expression of SmIMP25 at the RNA level according to Examples 3 and 5;

**Fig. 7** is a representation of an autoradiogram of a Southern blot of genomic DNA probed with 56HT and 63HT cDNA probes according to Example 4;

**Fig. 8** is a schematic representation of SmIMP25 cDNA fragments cloned into the pATH expression vector according to Example 8;

**Fig. 9** is a representation of an SDS-PAGE gel stained with Coomassie Brilliant Blue illustrating expression of the 134H-TrpE fusion protein in *E. coli* according to Example 8;

**Fig. 10** is a representation of a SDS-PAGE gel stained with Coomassie Brilliant Blue illustrating the enrichment of the 222H-TrpE fusion protein, according to Example 8; and

**Fig. 11** is a representation of the immunofluorescence staining of the surface of schistosomula by anti-134H-TrpE antibodies, according to Example 10.

## DETAILED DESCRIPTION OF THE INVENTION

The invention will now be described in more detail in the following non-limiting Examples and their accompanying Figures.

EXAMPLE 1:

The life cycle of *S.mansoni*

The life cycle of *S. mansoni* is presented schematically in Fig. 1.

A Puerto Rican strain of *Schistosoma mansoni* was used and was maintained by passage through *Biomphalaria glabrata* snails and outbred ICR mice. About eight *miracidia* were used to infect a single snail in water. Six weeks after infection *cercariae* were collected from infected snails that were kept in darkness. At four days intervals snails were exposed to bright light for 1 hr, yielding 1000-3000 cercariae/snail. To obtain adult worms, 300 cercariae were injected subcutaneously to one mouse, seven weeks later the mice were sacrificed and liver perfusion was done to yield 20-50 worms/mouse.

EXAMPLE 2: - GENERAL TECHNIQUES

(a) Preparation of schistosomula

Schistosomula were prepared from cercariae by mechanical transformation as described by Levi-Schaffer et al (1982), with a few modifications. About 400 ml of water containing cercariae were divided into 50 ml conical glass tubes. DSM (defined synthetic medium, 1 ml) the composition of which is detailed in Example 2(b)(i) below, was gently layered on the bottom of the tubes that were kept on ice for 30 minutes to reduce motility of the parasite. The tubes were centrifuged for 1500 rpm for 3 min in a clinical centrifuge. The pellet of cercariae in DSM was taken up by a 10 ml syringe to which another syringe was connected. The cercariae were subjected to shearing forces created by 20 passages between the syringes. This caused body-tail separation. A mixture of bodies and tails of approximately 100,000 cercariae in DSM (1 ml) were gently loaded on top of a 58% Percoll solution (7 ml) in 15 ml plastic tubes. The tubes were gradually centrifuged to 1500 rpm for 5 min. The tails (on the top of the step gradient) were separated from the bodies (in the bottom of the gradient). Bodies that yielded *schistosomula* were washed 5 times with DSM and cultured in costar plates (10,000/well) containing 1 ml DSM. Incubation was at 37°C in a $CO_2$ incubator, usually for 3 hours, and as needed up to 24 hours.

(b) Media:

(i) DSM (defined synthetic medium) is a 1:1 mixture of RPMI-1640 and F-12 media (GIBCO, Grand Island, NY, USA). Before use DSM was supplemented with L-glutamine (2 mM), Hepes buffer pH 7.2 (20 mM) and antibiotics (penicillin 100 U/ml, streptomycin 100 $\mu$g/ml). The 58% Percoll solution for body-tail separation was prepared from 58 ml Percoll (Pharmacia), 6 ml of RPMI-1640 concentrated x 10, 36 ml of DSM, and 1-2 drops of 5N HCl to adjust to pH 7.2. Adult worms were cultured in a medium composed of DSM and human serum in a 1:1 ratio.

(ii) Media of bacterial cultures and plates for the screening and preparation of recombinant phages and plasmids, as described in the following Examples, were used as described (Maniatis et al., 1989, pages A.1-A.7).

(c) Preparation of RNA

Total RNA was isolated from the various developmental stages of *S. mansoni* (Examples 1 and 2(a)) and organs of the snail host *B. glabrata* as described (see Ram et al., 1989). Poly(A)-rich RNA was obtained by passing total RNA on oligo(dT)-cellulose (BRL). The column was first washed with 20 mM NaOH and then with several volumes of loading buffer (0.5 M NaCl, 20 mM Tris pH 7.4, 0.1% SDS) until the pH was neutral. Total RNA was loaded, the column was washed with several column volumes of loading buffer, poly(A)-rich RNA was eluted in 5 mM Tris pH 7.4, 0.05% SDS, was subsequently alcohol precipitated, slightly dried and dissolved in sterile distilled water.

(d) Translation of mRNA and Immunoprecipitation of cell-free products

(i) **Translation**. Total RNA (Example 2(c)) was translated in the wheat germ cell-free extract (see Ram et al., 1989). A standard reaction of 40 $\mu$l contained: 5 $\mu$l wheat germ extract, total RNA (1-3.5 $\mu$g), 37.5 $\mu$M spermidine, 15 $\mu$g/ml creatine phosphokinase, 2 mM DTT, 30 $\mu$M 20 amino acids without methionine, 1.5 $\mu$l $^{35}$S-methionine (Amersham, 10 $\mu$Ci/$\mu$l, >800 Ci/mmole), 1.1 mM ATP, 0.25 mM GTP, 8.8 Mm creatine phosphate potassium salt, 20 mM Hepes pH 7.6, 6 mM Mg-acetate and 87.5 mM K-acetate. Reaction mixtures were incubated at 25 °C for 2 hr, and $^{35}$S-methionine incorporation into TCA precipitable material was measured.

(ii) **Immumprecipitation.** The above $^{35}$S-Met labelled cell-free translation products (500,000 cpm of TCA insoluble material) were preadsorbed with formalin inactivated *Staphylococcus* aureus on ice for 30 minutes in 0.3 ml of buffer A (50 mM Tris pH 7.4, 5 mM EDTA, 150 mM NaCl, 0.5% NP-40) supplemented with: aprotinin (1:100, Sigma), 1 mM methionine, 5 $\mu$l rat kidney lysate (2 mg/ml), and 25 $\mu$l of washed formalin inactivated *Staphylococcus aureus*. The mixture was centrifuged for 40 seconds and the pellet discarded. The first antibody (1 to 2 $\mu$l of rabbit antiserum) was added to the clear supernatant and the reaction was kept on ice overnight. Protein A Sepharose (3 mg, Pharmacia) in 80 $\mu$l of buffer A were added, and the reaction was kept on ice for 30 minutes (before use the Protein A Sepharose beads were suspended in buffer A, precipitated in an Eppendorf™ centrifuge, resuspended in PBS containing 5 mg/ml BSA and after agitation for 30 min at room temperature the beads were washed twice with buffer A). The reaction was centrifuged for 10 seconds to precipitate the beads. The beads were washed three times in buffer B (5% sucrose, 10 mM Tris pH 7.4, 500 mM NaCl, 5 mM EDTA, 0.5% NP-40) and once in buffer C (10 mM Tris pH 7.4, 150 mM NaCl, 5 mM EDTA). The protein products were removed from the beads, reduced, denatured and separated on SDS-PAGE as described (see Ram et al., 1989).

(e) DNA preparations

(i) **Parasite DNA**. High molecular weight DNA (Example 4) was prepared from worm nuclei as described (see Ram et al., 1989). Since it was difficult to isolate nuclei from cercariae, intact cercariae were used to prepare DNA. Cercariae were suspended in TNE buffer (10 mM Tris pH 7.5, 150 mM NaCl and 1 mM EDTA), SDS was added to 0.7% and the solution became viscous. Pronase was added to 0.2 mg/ml and incubation at 37 °C for 4 hr. The solution was extracted several times with an equal volume of phenol - 0.1% hydroxyquinoline (equilibrated with 0.2 M Tris pH 8) until no proteins were detected in the interface, and then once with chloroform. Two volumes of ethanol were added to the aqueous phase, the DNA was immediately spooled, washed in 70% ethanol, and dissolved in 5 mM Tris pH 7.4 - 0.5 mM EDTA. Gel electrophoresis showed genomic DNA to be larger than the 50Kb DNA marker.

(ii) **Phage DNA**. $10^7$ - $5 \times 10^7$ phage stock (of any of the phages used as noted in Examples 3 and 4) was adsorbed to 0.3 ml LE392 host bacteria (fresh overnight culture suspended in 5 mM MgCl$_2$ - 5 mM CaCl$_2$ - 5 mM Tris pH 8) at 37 °C for 20 min, and then inoculated into 400 ml of standard culture medium - NZYCM. The culture was incubated at 37 °C for about 8 hr with moderate agitation. When lysis appeared CHCl$_3$ (2 ml) was added and the culture agitated at 37 °C for 10 min to lyse all cells. NaCl (60 gr/l) was added and the agitation continued until NaCl was completely dissolved. Cell debris was spun down at 6000 rpm for 5 min in a GSA rotor. Precipitation of phages with polyethylene glycol 8000 (Sigma), purification of phage particles in cesium chloride gradient, and extraction of DNA, were performed according to Maniatis et al. (1989, pages 2.73-2.76). Yield of phage DNA was 100-200 $\mu$g from 200 ml culture.

A rapid procedure was also developed to prepare small quantities of the above phage DNA (1-5 $\mu$g) within 3 hours. Phage infected bacteria (5-6 hr culture) were lysed with chloroform. 0.75 ml of the lysate and 0.5 ml of DE-52 slurry (Whatman) equilibrated in LB-Mg broth, were mixed in an Eppendorf™ tube. DE-52 beads were spun down by centrifugation for 30 seconds, SDS and EDTA were added to the supernatant to final concentrations of 0.5% and 25 mM, respectively, and incubation at 68 °C for 10 min. Tris pH 8 and proteinase K (Sigma) were added to final concentrations of 100 mM and 50 $\mu$g/ml, respectively. After 30 min at 37 °C the reaction mixture was extracted with phenol-chloroform until the interface was clear, then precipitated with equal volume of isopropanol for 15 min at 4 °C, washed twice with 70% ethanol and the phage DNA dissolved in 50 $\mu$l of TE. Restriction analysis was done on 5 $\mu$l aliquotes. 18 samples could be processed simultaneously within 3 hr to provide clear DNA digestible to completion with restriction enzymes, and suitable for subcloning of the DNA inserts into plasmid vectors.

(iii) **Plasmid DNA**. Plasmids (being any of those used in Example 6) were grown in HB101 bacteria that were lysed by lysozyme. Plasmid DNA was purified by equilibrium centrifugation in cesium chloride-ethidium bromide to yield 0.2-1 mg of plasmid from 400 ml culture. Minipreparations of plasmid DNA from 3 ml culture, sufficient for 5-10 reactions of restriction enzyme analyses, were done according to the alkaline lysis method.

(f) DNA nucleotide sequencing

Nucleotide sequencing was done by the dideoxy chain termination method (see Ram et al., 1989). All DNA nucleotide sequences were determined at least twice.

(g) Primer extension experiments

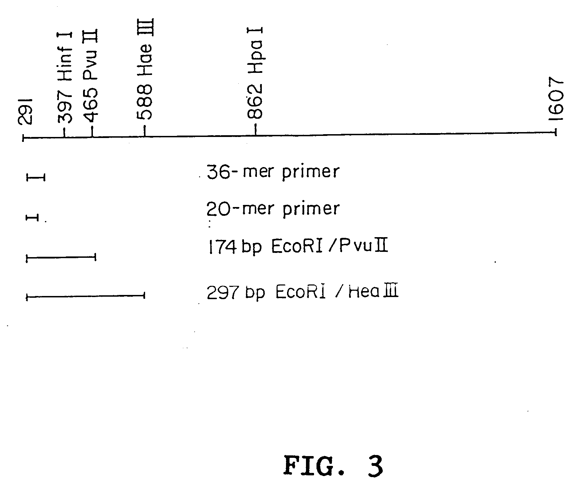

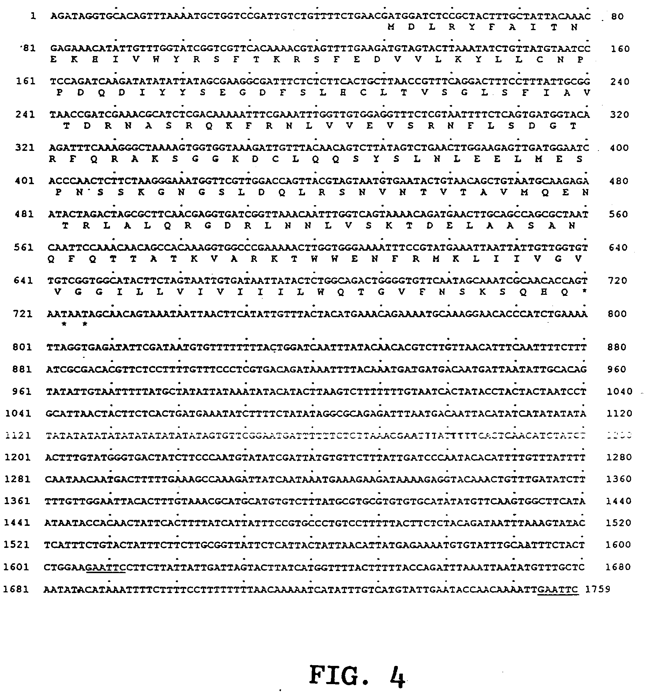

Two oligonucleotide primers close to the 5' end of 63HT (Examples 3-5) were synthesized (Fig. 3). The 36-mer oligonucleotide (5'-CCACTTTTAGCCCTTTGAAATCTTGTACCATCACTG-3') was complementary to the cDNA sequence between positions 343 to 308, and the 20-mer oligonucleotide (5'-GTACCATCACTGAGAAAATT-3') was complementary to the cDNA region 319 to 300 (see Fig. 4). The primer was end labelled by polynucleotide kinase (NEN Research Products) and $^{32}$P $\gamma$-ATP. The labelled primer ($2 \times 10^6$ cpm) was hybridized for 1 hr at 56°C to either 100 $\mu$g total RNA of adult worms or 5 $\mu$g of poly(A)-rich RNA (Example 2(c)) in 250 mM NaCl - 25 mM Tris pH 7.5. The solution was adjusted to 500 mM NaCl - 0.1% SDS and purified over oligo(dT)-cellulose column (100 $\mu$l bed volume) to remove non-poly(A)-rich RNA and non-annealed primer. The remaining bound poly(A)-rich RNA, which is essentially mRNA that is hybridized to the primer was then eluted with 10 mM Tris pH 7.5 and 0.05% SDS. The eluted mRNA-primer hybrid was ethanol precipitated and dissolved in 15 $\mu$l 10 mM Tris pH 7.5 and 1 mM EDTA. Reaction mixtures contained the mRNA-primer hybrid, reverse transcriptase (Molecular Genetic Resources) and either dNTP's for size determination of the extended product, or dNTP's and dideoxynucleotides for sequence analyses. Reaction products were analyzed on 8% sequencing gel.

(h) mRNA hybrid selection

DNA (5 $\mu$g) of a pBR322 recombinant (prepared according to Example 2(e)(iii)) was linearized by restriction enzyme digestion, it was denatured by heating (95°C/5 min), rapidly chilled, applied to a Hybond-N filter (Amersham) and U.V. irradiated to fix the DNA. Hybridization of the DNA to 50-100 $\mu$g parasite total RNA (Example 2(c)) and elution of the hybridized RNA were performed as described (see Ram et al., 1989). The eluted RNA was translated in the wheat germ extract and the protein products immuno-precipitated by relevant antisera (procedure according to Example 2(d)(ii)).

(i) Southern and Northern blots

(i) **Southern blots** were done as described (see Ram et al., 1989), with few modifications. High molecular weight genomic DNA (Example 2(e)(i)) was digested with one or more restriction endonucleases (New England Biolabs) in buffers recommended by the supplier. The DNA digests were separated by electrophoresis on horizontal 0.8%-1% agarose gels (Seakem) containing 0.5 $\mu$g/ml ethidium bromide in TBE buffer (0.09 M Tris, 0.09 M boric acid and 2 mM EDTA, pH 7.8). DNA bands were visualised by U.V. illumination. The DNA fragments in the gel were slightly depurinated by soaking the gel in 0.2 N HCl for 15 min (twice). The DNA in the gel was denatured by transferring the gel into 0.5 N NaOH for 15 min (twice), then neutralized by soaking the gel in 20XSSC and titering to neutrality with a solution of 1 M Tris base and 1.5 M HCl. The DNA was then transferred from the gel to Hybond-N filter (Amersham) in 20XSSC over 3 hr, it was washed in 6XSSC and baked in vacuum at 80°C for 1 hr. The filter was prehybridized, hybridized, washed and exposed to X-ray film as described in Example 6.

(ii) **Northern blots** were done as described (see Ram et al., 1989), with a few modifications. 5-10 $\mu$g total RNA (Example 2(c)) in 6 $\mu$l were denatured by the addition of 18 $\mu$l of denaturation solution: 640 $\mu$l formamide, 230 $\mu$l formaldehyde and 130 $\mu$l 10xMOPS buffer (400 mM MOPS, 100 mM sodium acetate and 10 mM EDTA pH 7.2). The samples were heated for 10 min at 60°C. 6 $\mu$l of loading dye solution (20% Ficoll, 0.1% bromophenol blue) were added and the sample was loaded on a horizontal 1% agarose gel containing 2.2 M formaldehyde in 1xMOPS buffer. Electrophoresis was at 70 volts till the dye migrated 8-10 cm from the origin (~7 hr). The gel was blotted overnight with 10xSSC (1.5 M NaCl, 0.15 M Na$_3$ -citrate) onto a Hybond-N filter. The filter was baked for one hour at 80°C in vacuum to fix the RNA. The filter was prehybridized at 42°C for 4 hours in 5xSSC, 1xDenhardt's (0.02% BSA, 0.02% Polyvinyl Pyrolidone, 0.02% Ficoll), 50% formamide, 20 mM Na-Pi buffer pH 6.8, 25 mM EDTA, 0.1% SDS, and 100 $\mu$g/ml sheared and denatured salmon sperm DNA. Hybridization for 16 hours at 42°C was done in the same solution to which was added $0.5-1 \times 10^6$ cpm/ml $^{32}$P-labelled DNA probe (Example 2(j)) denatured by boiling for 6 min. After hybridization the blot was first washed at 42°C with a solution containing 2xSSC, 0.05% SDS, 20 mM NaPi buffer pH 7.0 and 1.5% NaPiPi, and then washed at 68°C in the same solution as described in Example 5. The filter was exposed to X-ray film with an intensifying screen at -70 ° C.

(j) Preparation of $^{32}$P labelled probes - nick translation and multiprime labelling

Nick translation was performed as described (see Ram et al. 1989). 50 to 200 ng of DNA, to be used as a hybridization probe as described below in the following Examples, were incubated at 15 ° C with 50 $\mu$ Ci of one or two $^{32}$P dNTP(s) (3000 Ci/mmole), 10 $\mu$M of the other cold nucleotides and 5 units of *E. coli* polymerase I (New England Biolabs) in 50 $\mu$l of buffer containing 40 mM Tris pH 8.0, 5 mM MgCl$_2$, 0.5 mg/ml BSA, 5 mM dithiothreitol and 1 mM spermidine. The incubation was continued for about 15 min until 20-40% of the radioactivity was incorporated into TCA insoluble material. The reaction was stopped by the addition of 200 $\mu$l of TE (10 mM Tris pH 8.0, 10 mM EDTA) containing 0.5% SDS, and the labelled DNA was separated from the free nucleotides by chromatography on Sephadex G-50™ column. Subsequently multiprime DNA labelling was performed with a multiprime labelling system (Amersham) in which $^{32}$P incorporation was 60-95%.

EXAMPLE 3:

Worm cDNA expression library and screening with antibody probe for cDNA isolation

The poly(A)-rich RNA of adult worm (Example 2(c)) was used to construct a cDNA library of the λgt11 expression vector (for vector DNA preparation see Example 2(e)(ii) above) as described (see Ram et al., 1989). The number of independent recombinant phages was 700,000 (white plaques) comprising 71% of the total PFU (white + blue plaques). The original library was amplified and stored in TMG buffer (10 mM Tris pH 8.0, 10 mM MgCl$_2$ and 0.1% gelatin) at 4 ° C till used. The cDNA library was screened with antisera of rabbits immunized with tegumental surface membranes for 3 hr schistosomula. Briefly, the surface tegument was isolated from 3 hr mechanical schistosomula by hypertonic osmotic shock and mechanical shearing as described (Levy-Schaffer et al., 1984). Rabbits were immunized with the membrane preparation (250 $\mu$g/injection) emulsified in complete Freund's adjuvant. Three intradermal injections were administered at two weeks intervals, followed by two injections after 2 months and 6 months. Antisera were collected 10 days after the 5th injection. The antisera (1:100 dilution) were used to screen the λgt11 expression library (30,000 PFU/plate) according to standard procedures (see Ram et al., 1989).

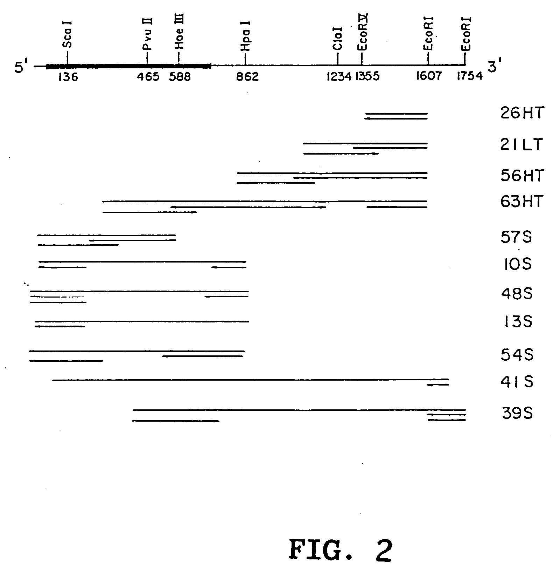

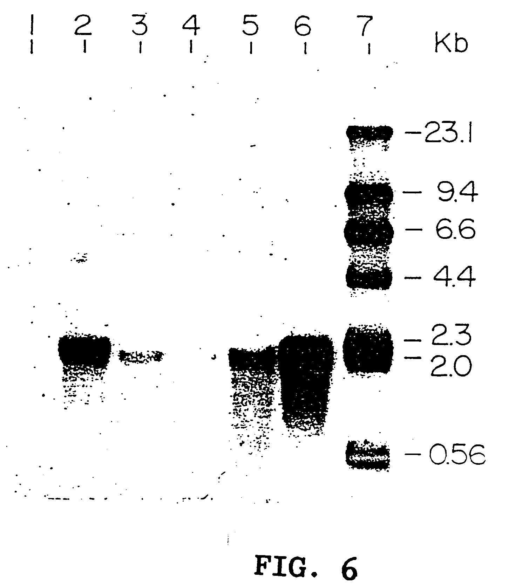

Seven clones were isolated from the above worm cDNA library constructed in the λgt11 expression vector by screening with the above sera. Two clones, 26HT (0.27 Kb) and 21LT (0.5 Kb), cross-hybridized with each other, showed identical hybridization patterns in Southern blots and detected the same mRNA in Northern blots (see Example 2(i)(i) and (ii) for details of blot procedure). Since the cDNAs were smaller than the mRNA detected in Northern blots (2 Kb, see Fig. 6) additional clones were sought. For this purpose the 21LT insert was subcloned by standard procedures into pBR322 and used as a probe to screen the worm cDNA library by molecular hybridization (for procedure see Examples 5 to 7). Two such additional clones were isolated: 56HT (0.8 Kb) and 63HT (1.3 Kb). Alignment of the clones revealed that a substantial portion of the corresponding mRNA from which these cDNA clones were originally derived, was still missing. The 5' region was further investigated because only the 5' portion of the above clone 63HT had an open reading frame of significant length, encoding 143 amino acids (see Fig. 2).

To determine the above 5' end of the mRNA oligonucleotides were synthesised complementary to the 5' end of 63HT cDNA (Fig. 3) for primer extension experiments using adult worm RNA (for details of procedure see Example 2(g)). Of these the 20-mer oligonucleotide primer proved unsuitable, i.e., extended product was not formed. The longer 36-mer oligonucleotide, however, proved suitable possibly since fairly long primers are often needed for extension experiments using total RNA (or poly(A)-rich RNA), particularly when the mRNA is of low abundance. Primer extension with this 36-mer oligonucleotide showed a few bands of 280-380 bases suggesting that the mRNA is at most 330 bases longer than 63HT (in 5' direction), or 40 bases longer than the composite sequence given in Figs. 2 and 4. As depicted in Fig. 3, a restriction map of the 63HT cDNA clone was made. The DNA probes derived from this cDNA clone and the synthetic oligonucleotide primers complementary to parts of this cDNA are also presented. The various pertinent restriction sites are indicated and it should be noted that the EcoRI site at position 291 is from the linker used to construct the cDNA library while that at position 1607 is an authentic EcoRI site of the cDNA. Sequencing of the primer extension products was unsuccessful, possibly because the mRNA is of low abundance as it encodes a membrane protein that usually is present in minute amounts, and/or due to structural features of the mRNA. Thus, the worm cDNA library was rescreened with the EcoRI/HaeIII fragment (297 bp) from the 5' end of 63HT (Fig. 3). Several clones were isolated and seven of them were further charcterized: 57S (0.55 Kb), 10S (0.83 Kb), 48S (0.95 Kb), 13S (0.85 Kb), 54S (0.86 Kb), 41S (1.65 Kb) and 39S (1.5 Kb), as shown in Fig. 2.

The aforesaid clone 63HT is 1315 bp long but only 429 bases have an open reading frame encoding 143 amino acids, and a computer search did not reveal significant homology to known proteins. Therefore, it was important to determine whether the coding strand of the clone is expressed. For this purpose adult worm RNA was hybridized with the two complementary strands of 63HT cloned in M13. The single strand specific probes were prepared from two M13 recombinants containing the 63HT sequence in opposite orientation. The sequence labelling procedure without dideoxynucleotides was used to label the M13 recombinant DNA, the reaction product was digested with EcoRI, separation of the labelled probe (1.3 Kb) from the residual M13 phage (7.2 Kb) by electrophoresis on 5% acrylamide gel, and elution of the probe. The probe of the antisense strand hybridized to the adult worm mRNA while the probe of the coding strand showed no hybridization. This finding established that the coding strand of the 63HT cDNA clone is expressed in the parasite. In addition, a protein encoded by the open reading frame (synthesized in bacteria as TrpE-fusion protein, see Example 8) elicited antibodies that immunoprecipitated the expected protein programmed *in vitro* by schistosome RNA, demonstrating that the open reading frame of clone 63HT is transcribed into translatable mRNA and accordingly it represents an expressed gene that encodes a protein which, as will become more apparent hereinbelow, was designated SmIMP25, because this protein is an integral membrane protein of mol. wt. of about 25 KD (unglycosylated). As is summarized in Fig. 2, the coding region of SmIMP25 cDNA is marked by a thick line and the untranslated regions by thin lines. The arrows indicate the direction and extent of sequencing of each of the various above-noted cDNA clones. The various restriction enzyme sites used for characterizing and aligning these cDNA clones is also presented. The nucleotide numbering is according to the sequence of Fig. 4. The sequence of Fig. 4 is in fact a composite of the sequences of the various cDNAs shown in Fig. 2. It should be noted in Fig. 4 that there are three consecutive termination codons at the end of the coding region which are indicated by asterisks. The EcoRI sites at positions 1607 and 1754 are underlined.

## EXAMPLE 4:

### Genomic DNA libraries

Genomic DNA libraries of cercariae were constructed in the EMBL4 phage vector according to standard procedure (see Ram et al., 1989). Cercarial DNA (for DNA preparation see Example 2(e)(i) above) was partially digested with Sau3A, the digest was run on 0.8% agarose gel, the high molecular weight fraction (15Kb - 23Kb) was electroeluted from the gel and ligated to the arms of the EMBL4 phage vector. After packaging the recombinant phages were used to transfect *E. coli* LE392 host cells. The number of independent recombinants was $2.6 \times 10^5$ comprising 89% of total PFU. The library was amplified in *E. coli* LE392 host cells to a titer of $3 \times 10^9$ pfu/ml.

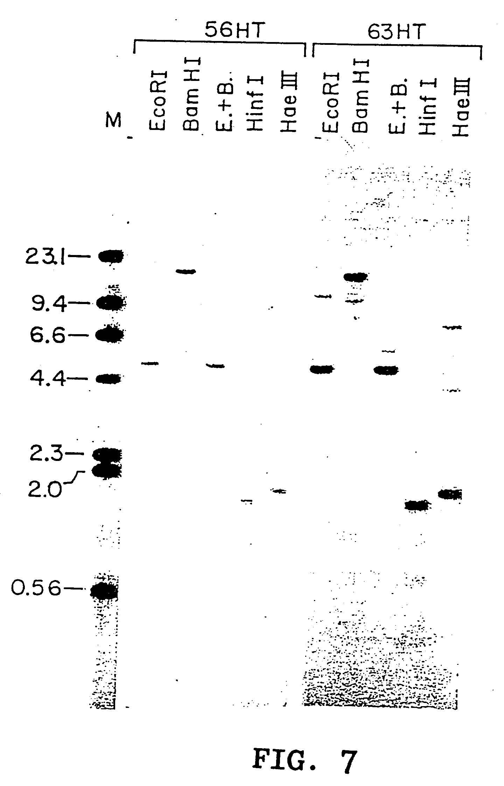

For the characterization of the above cercariae genomic DNA libraries, uncloned genomic DNA of *S. mansoni* (prepared according to Example 2(e)(i) above) was digested with several restriction enzymes including EcoRI, BamHI, Hinfl, HaeIII (New England Biolabs, digestion conditions according to supplier's instructions, and hybridized with the 56HT cDNA (Example 3). The Southern blot (procedure according to Example 2(i)(i) above) showed a simple pattern of one hybridizing band for each restriction enzyme. The same digests hybridized with the 63HT cDNA (Example 3) revealed additional bands (Fig. 7), indicating the existence of introns in that portion of the gene encoding the 5' region of 63HT. For example, the 63HT cDNA has no EcoRI site and one HaeIII site close to the 5' end, yet in the Southern blot the probe hybridized with two EcoRI and three HaeIII fragments (see Figs. 2 and 7). Clone 57S (Example 3) with an additional 265bp upstream of the 5' end of 63HT (see Fig. 2) showed the same hybridization pattern as 63HT clone, indicating that the 5' 265bp are located on the 10.5kb EcoRI genomic fragment detected by the 63HT cDNA (see Fig. 7), as subsequently found in the cloned gene.

Thus, in view of the above results, the aforesaid genomic library of cercarial DNA (partial Sau3A digest) constructed in EMBL4 was screened with the 63HT cDNA probe. Eight clones were isolated. EcoRI digests of the clones were separated on agarose gels and hybridized with the 63HT and 56HT probes. Two clones (6 and 9) had 5 Kb EcoRI fragments hybridizing with the two probes. This fragment matches with the 5 Kb EcoRI fragment revealed in the genomic DNA digest (Fig. 7) and corresponds to the 3' region of the gene. The remaining six clones hybridized only with 63HT, indicating that they contain fragments from the 5' portion of the gene. These clones (designated 18, 19, 20, 22, 25 and 30) were digested with Hinfl and HaeIII that cleave at the 5' end of 63HT. The digests were separated on agarose gel and hybridized with three different probes derived from the 5' end of 63HT (see Fig. 3): 1) A 20-mer synthetic oligonucleotide (Examples 2(g) and 3) corresponding to the very 5' end of 63HT - denoted as the 5' end probe; 2) The 5' 174 bp EcoRI/PvuII fragment of 63HT - denoted as the PvuII probe; 3) The 5' 297 bp EcoRI/HaeIII fragment of 63HT - denoted as the HaeIII probe. Blots of the clones and of the genomic DNA (Fig. 7) enabled the identification of cloned fragments identical to the genomic ones (i.e. not digested by Sau3A at the ends) and provided some information on their genomic organization. Because the fragments were fairly long for sequencing they were further characterized. The 10.5 Kb EcoRI fragment from clone 20 was subcloned into pBR322, a physical map was constructed based on restriction enzyme digests hybridized with the various 63HT derived probes (Fig. 3). The positive fragments were identified, subcloned into the M13 vector and sequenced (procedure according to Example 2(f)). Clone 9 encoding the 3' portion of the gene was digested with HaeIII. The 1.8 Kb HaeIII fragment hybridized with 3' probes of the cDNA and its size was identical to the 1.8 Kb HaeIII fragment revealed in the Southern blot (Fig. 7). The 1.8 Kb HaeIII fragment was cloned, it was further digested with RsaI, subcloned into the M13 vector and sequenced. It was found to contain two EcoRI fragments of 147 bp followed by a 232 bp fragment at the 3' region of the gene. The sequence of the 147 bp EcoRI fragment was identified in the cDNA (Figs. 2 and 4).

EXAMPLE 5:

Screening of phage libraries by molecular hybridization

A few cDNA clones encoding part of the above protein, designated SmIMP25 (Examples 3 and 4) were isolated from the λgt11 expression library of worm cDNA by screening with antisera against the tegument of schistosomula (Example 3). To obtain the full length cDNA additional clones were isolated from the λgt11 library by molecular hybridization using cloned cDNA's as DNA probes (Example 2(j)). cDNA probes were also used to screen the EMBL4 genomic library (Example 4) to clone the SmIMP25 gene. Screening was done according to standard procedures (see Ram et al., 1989), with some minor modifications. Briefly, nitrocellulose filters were layered on the plates (35,000 PFU/plate) for 3 min (1st filter) and 6 min (2nd filter). The filters were subjected to denaturation by adding thereto a solution containing 0.2 M NaOH and 1.5 M NaCl for 2 min followed by neutralization with a solution containing 0.5 M Tris pH 7.4 and 3 M NaCl, for 5 min which was repeated. The filters were dried in vacuum at 80°C for 1 hour, and washed twice with 6XSSC buffer containing 0.08% Ficoll at 68°C for 15 min. The prehybridization and hybridization were carried out in a solution containing 6XSSC, 0.08% Ficoll, 0.02% PVP, 0.02% BSA and 0.1% SDS. The DNA probe prepared according to Example 2(j) (500,000 cpm/ml, 2-10 ng/ml) and salmon sperm DNA (50 μg/ml) were boiled for 5 minutes and rapidly chilled before adding to the hybridization solution. Prehybridization was for 3-4 hours at 68°C, and hybridization was for 20 hr in a shaking water bath at 68°C. The filters were washed at 68°C with a 2XSSC, 0.05% SDS and 20 mM NaPi buffer solution of pH 7.0 containing 1.5% NaPiPi, until radioactivity in the washing solution was markedly low, then 1-2 washes in the same solution diluted in water 1:4 and 1:10. The filters were dried and exposed to X-ray films with an intensifying screen at 70°C. Positive plaques were purified by repeated plating, and stored in TMG with a drop of chloroform at 4°C.

The inserts of the λgt11 cDNA clones which were isolated following above screening procedure (shown in Fig. 2) were further subcloned into the KS (Bluescript™) and/or M13 vectors, and sequenced (sequencing method according to Example 2(f)). Alignment of the clones (Fig. 2) has led to a composite sequences of 1759 bp of the cDNA, termed SmIMP25 (Fig. 4). The cDNA has an open reading frame of 669 bases encoding 223 amino acids, starting with ATG (position 51) inserted in the ACG ATGG sequence (see Fig. 4) most frequently found for the initiator- Met codon of eukaryotes (PuNNATGG), and ending with three termination codons TAA-TAA-TAG (position 720). The apparent 5' untranslated region is 50 bases long. The transcription initiation site was not determined, however the primer extension products indicate at most 40 additional bases in the 5' direction. The 3' untranslated region (downstream from position 72)) is 1040 bases long with many termination codons. The poly(A)-tail was not found, probably because the 3' end contains EcoRI sites used for cloning the cDNA. The 39S cDNA clone contains a 3' 147 bp EcoRI fragment (see Figs. 2 and 4) that matches with the gene. In the gene, the 3' end of this 147 bp EcoRI fragment is linked to another 232 bp EcoRI fragment (not shown). Clone 39S seems to contain at least a portion of this 232 bp sequence, since it hybridized with the 232 bp EcoRI genomic fragment (data not shown), but this segment was not yet identified in the 39S cDNA. Considering the sizes of the cDNA and mRNA, we estimate that the 3' untranslated region may be about 200 bases longer. The nucleotide sequence of the cDNA and of the gene introns excluded (see Example 4) showed 100% identity.

Thus, the open reading frame of the above SmIMP25 gene encodes a protein 223 residues long with mol. wt. (calculated from the amino acid sequence) of 25,381 daltons. The predicted amino acid (a.a.) sequence reveals three putative N-glycosylation sites (Asn-X-Thr/Ser) and a hydrophobic domain of 20 a.a. located 12 residues from the carboxyl terminal end (Fig. 5). The predicted amino acid sequence of SmIMP25 shown in Fig. 5 was deduced from the nucleotide sequence of Fig. 4 as determined according to the above methods. The underlined amino acids in Fig. 5 denote the three potential N-glycosylation sites at positions 68-70, 119-121 and 124-126 and the hydrophobic transmembrane segment at position 192-211. The hydrophobic domain is composed of hydrophobic residues (Ile, Leu, Val, Gly and Trp), and it is flanked by charged residues (Lys, Arg, His). The properties of this domain (Marked hydrophobicity, size, flanking by charged residues and C-terminal location) are typical of transmembrane segments of integral membrane proteins (e.g. receptors). The presence of three glycosylation sites is also consistent with integral membrane proteins that are often glycosylated.

Therefore it is concluded that the SmIMP25 gene encodes an integral membrane protein, in which residues 1-191 would face the outer cell-surface, residues 192-211 are embedded in the cell membrane, and residues 212-223 are intracytoplasmatic. The transmembrane segment may be longer, consisting of 25 residues from Leu-192 to Phe-216 (Fig. 5). The hydrophobicity plot (not shown) shows the transmembrane segment, as expected. In addition, it shows that the N-glycosylation sites coincide with peaks of negative hydropathic index, indicating four hydrophilic regions exposed on the outer surface of the molecule. Computer search has not revealed significant homology between SmIMP25 and known proteins, including any of the published sequences of *Schistosoma* proteins.

Furthermore, as revealed by the above analysis, the SmIMP25 gene is at least 16 Kb long, and it is split by four introns ranging in size from 36 bp to 9 Kb. The splice signals that begin and end the intron, as well as the pyrimidine-rich stretch near the 3' end of the intron are present in all four introns (Table 1), and conform with the consensus sequences (see Ram et al., 1989). The entire sequence of the cDNA is split into five exons located on three EcoRI genomic fragments.

## TABLE 1

### Consensus sequences of the 5' and 3' splice signals in introns of the SmIMP25 gene.

| | 5' | | | 3' | | |
|---|---|---|---|---|---|---|
| consensus sequence | A A G | G T | A A G T | pyrimidine rich sequence | A G | G |
| Intron I | C G A | G T | A A G T | T C A T T T T C A C T C T | A G | T |
| Intron II | G A G | G T | A A G T | C A C T T T A T T C T T C | A G | G |
| Intron III | A T G | G T | A A G T | T T T A C T G T A T T T C | A G | G |
| Intron IV | A G C | G T | G A G T | A A C G T T T T T C T T C | A G | G |

To evaluate whether the expression of the above SmIMP25 gene is developmentally regulated the RNAs of miracidia, sporocyst, cercaria and adult worm were analysed. The sporocysts reside in the hepatopancreas (HP) of the snail where they multiply asexually and differentiate to cercariae. In infected HP about 50% of the RNA is parasite specific. Routinely, the infected snails are triggered to shed cercariae at 4 day intervals by exposure to bright light. RNA was extracted from infected snails at two time points: 1) HP of infected snails immediately after cercarial shedding that is enriched for sporocysts, denoted as early sporocysts; 2) HP of infected snails 4 days after shedding containing many cercariae ready to emerge as well as sporocysts, denoted as late sporocysts. RNA from the HP of uninfected snails and mouse kidney served as controls. Northern blots showed hybridization of the cDNA probe with a 2 Kb mRNA present in all developmental stages, the hybridization was stronger in worm and miracidia than in cercaria and sporocyst. The mouse kidney RNA and the uninfected HP, which served as controls, did not hybridize with the 63HT cDNA (Fig. 6). As depicted in Fig. 6 the above results can be observed in a Northern blot of total RNA (10 $\mu$g/lane) from the various life cycle stages of the parasite and hybridized with a $^{32}$P-labelled 63HT cDNA probe (Examples 2(j) and 3), where lane 1 is uninfected snail hepatopancreas RNA, lane 2 is miracidia RNA, lane 3 is early sporocyst RNA, lane 4 is late sporocyst RNA, lane 5 is cercaria RNA, lane 6 is adult worm RNA and lane 7 is DNA size markers.

Thus, in view of the above finding that the SmIMP protein is expressed in all developmental stages of the parasite, it can be concluded that this protein is a good candidate for use as a vaccine. This is shown to be in fact the case as set forth below.

### EXAMPLE 6:

Transfection of bacteria with plasmids

*E. coli* strains (HB101 and 522) were made competent for transfection by recombinants of plasmids pBR322 and pATH in strain HB101, and of Bluescript™ plasmid in strain 522 (see Example 8), as follows: Bacteria were grown in L-Broth to $A_{650}$ = 0.5, the culture was cooled and centrifuged 2000 rpm for 10 min at 4°C. Precipitated bacteria were resuspended in 1/3 volume of a solution 1 containing 100 mM RbCl, 45 mM $MnCl_2$, 10 mM $CaCl_2$, 35 mM K-acetate and 15% glycerol, the suspension was kept on ice for 90 minutes, centrifuged and the precipitated bacteria were gently resuspended in 1/25 volume of a solution 2 containing 10 mM MOPS pH 7, 10 mM RbCl, 75 mM $CaCl_2$ and 15% glycerol and quickly frozen in liquid nitrogen for a long period of storage (more than 1 year).However, when the above competent bacteria were prepared for immediate use the above resuspension was in half a volume of 0.1 M $CaCl_2$, kept on ice for 20 min, precipitated and resuspended gently in 1/10 volume of 0.1 M $CaCl_2$. Competent bacteria prepared in this way can be used for up to one day when kept at 4°C.

For the transfection procedure, either 50 $\mu$l of the above frozen or 300 $\mu$l of the above freshly prepared competent bacteria were mixed with 50 ng of recombinant plasmid DNA, kept on ice for 40 minutes, heat shocked at 37°C for 5 minutes or at 42°C for 2 minutes, LB broth (1 ml) was added, the suspension was incubated at 37°C for one hour and plated on agar plates containing 100 $\mu$g/ml ampicillin.

### EXAMPLE 7:

Screening of bacterial colonies transfected with plasmids

Bacteria transfected with recombinant plasmids (Example 6) were grown on agar plates at low density (1000-2000 bacteria/13 cm plate) to generate small colonies ($\leq$0.5 mm diameter). Nitrocellulose filters were layered on the plates for 5 min. The filters were subjected to denaturation (0.5 M NaOH for 7 min followed by 0.1 M NaOH and 1.5 M NaCl for 10 min) and neutralization (0.2 M Tris pH 7.4 and 2 mM EDTA for 2 min, followed by 1xSSC and 25 mM NaPi buffer pH 7 with 2 mM EDTA for 2 min, X 2). The filters were dried in vacuum at 80°C for 1 hr and washed twice with 0.1% SDS at 68°C for 15 min. Prehybridization and hybridization were performed as described in Example 6. Positive colonies were kept in LB broth (20%)- glycerol (80%) at -20°C.

### EXAMPLE 8:

Preparation of SmIMP25-TrpE and antibodies to the fusion protein

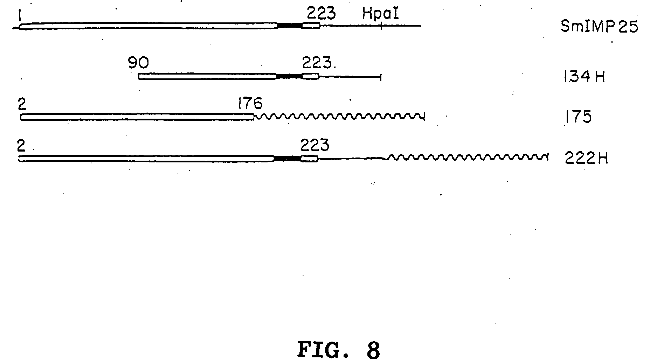

Three different cDNAs of SmIMP25 (Example 5) encoding 134 a.a., 175 a.a. and 222 a.a. (Fig. 8) were cloned into the TrpE gene of the pATH expression vector (Tanese et al., 1986) in the correct reading frame, as follows:

(i) 134H is the Rsal/Hpal fragment from the cDNA 63HT, it is 545 bp long (positions 317 to 862 in Fig. 4), and it was cloned into the Smal site of the pATH vector. The fragment encodes 134 amino acids (a.a.) from the C-terminus of SmIMP25, including the hydrophobic domain.

(ii) 175 was prepared by cloning the insert of cDNA 57S (Fig. 2) from λgt11 into the EcoRI site of pBR322. The recombinant plasmid was digested with XhoII which cuts at the 5' end of 57S (position 53 in Fig. 4) and at the BamHI site of pBR322 (position 375 in pBR322). The resulting fragment was composed of a segment derived from 57S (525 bp) and a segment derived from pBR322 (375 bp). This fragment was cloned into the BamHI site of the pATH vector. Many termination codons in all three reading frames are present in the pBR322 segment linked to the 3' end of the 57S segment. This DNA encodes 175 a.a. from the N-terminus of SmIMP25, without the hydrophobic domain.

(iii) 222H was prepared by cloning the insert of cDNA 10S (see Fig- 2) from λgt11 into pBR322, digestion of the recombinant plasmid with XhoII and cloning of the resulting fragment (809 bp of 10S plus 375 bp of pBR322) into the BamHI site of the pATH vector, similar to 175 above. This DNA encodes 222 a.a. corresponding to the full length native SmIMP25, including the hydrophobic domain, but without the initiator-Met residue that is naturally removed from the protein in the cell. As is summarized in Fig. 8, the SmIMP25 cDNA and fragments thereof cloned into the pATH expression vector are depicted as: Top - coding region and adjacent non-coding region of SmIMP25 cDNA and then the 134H, 175 and 222H fragments of SmIMP25 cDNA subcloned into TrpE gene of pATH. The numbers refer to amino acid codons where 1 is the initiator-Met residue and 223 is the C-terminal Gln residue (Figs. 4 and 5). The black box indicates the transmembrane region, the wavy line indicates a 375 base pair pBR322 fragment linker used during cloning.

All of the above recombinant plasmids and the non-transformed vector (as a control) were used to transform *E. coli* HB101 host cells (for plasmid DNA preparation and host cell transformation procedures see Examples 2(e)(iii) and 6).

The transformed bacteria were grown in 500 ml of M9 minimal medium supplemented with 1% casamino acids, 10 mM glucose and 100 $\mu$g/ml ampicillin, in a shaker at 37°C. After 3-4 hr of incubation when $A_{650}$ reached 0.2-0.3 OD, IAA (indole acrylic acid) was added to a final concentration 10 $\mu$g/ml to induce the TrpE gene, and the culture left overnight in the 37°C shaker. Bacteria were centrifuged, suspended in 100 ml of 50 mM Tris pH 7.4 - 5 mM EDTA - 3 mg/ml lysozyme, and kept on ice for 2 hrs. NaCl (to 0.3 M) and NP-40 (to 0.75%) were then added, bacteria were sonicated (tip limit 5, 60% duty cycle, pulses) four times each of 15 sec., and centrifuged (10,000 rmp/10 min).

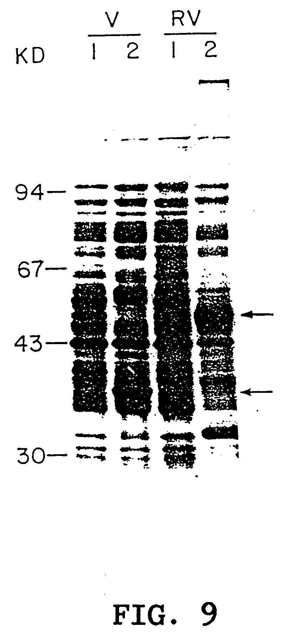

Analyses of the proper bacterial lysates before and after induction showed that bacteria harbouring recombinant plasmids expressed fusion proteins of correct size. For example, the 134H recombinant synthesized the expected 52KD fusion protein (37KD TrpE + 15KD fragment of SmIMP25), while the control plasmid vector expressed the 35KD TrpE protein (Fig. 9). The above results are apparent from Fig. 9 which represents an SDS-PAGE gel stained with Coomassie Brilliant Blue, from which it can be seen that the 134H-TrpE fusion protein was well expressed in the transformed *E. coli* HB101 host cells. Lysates prepared from non-induced (lane V 1) and induced (lane V 2) bacteria transfected with pATH vector alone, and lysates prepared from bacteria transfected with the 134H-TrpE recombinant vector (lanes RV 1, 2) are compared where the arrows indicate the positions of induced TrpE (lower arrow) and the induced 134H-TrpE fusion protein (upper arrow). Protein size markers are also indicated (sizes in KD).

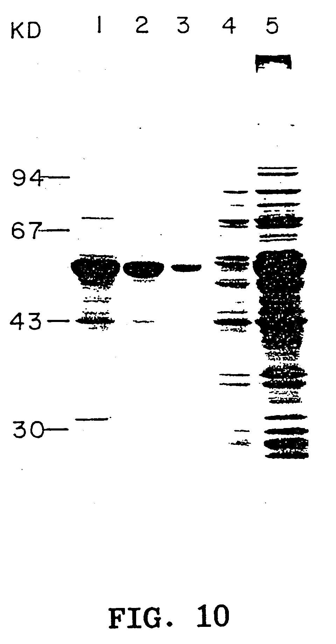

Fractions enriched with the fusion proteins were prepared by centrifugation of the whole bacterial lysate. The SmIMP25-TrpE fusion proteins were found in the precipitate because they were insoluble like many other TrpE fusion proteins. Therefore, the supernatant was discarded and the precipitate was dissolved in 20 ml PBS with added SDS (1%) and Triton X-100 (1%) to provide a preparation enriched with the SmIMP25-TrpE fusion protein. This preparation was then analysed on SDS-PAGE which, after staining of the gels, showed many proteins with a predominant band of the SmIMP25-TrpE fusion protein comprising about 15% of the total proteins and about 75% of the insoluble proteins (Fig. 10). As shown in Fig. 10, representing a SDS-PAGE gel stained with Coomassie Brilliant Blue, the above enrichment of 222H-TrpE fusion protein by centrifugation of the whole bacterial lysate was successful: lanes 1, 2 and 3 represent different aliquots of the lysate precipitate containing the fusion protein, lane 4 represents the lysate supernatant and lane 5 represents whole bacterial lysate. Estimated yield of SmIMP25-TrpE fusion protein was 20-30 mg from 200 ml culture.

For antibody preparation the precipitate, as noted above, from lysates of transformed bacteria, enriched with SmIMP25-TrpE fusion protein was dissolved in PBS-1% SDS-1% Triton X-100 and emulsified in complete Freund's adjuvant. As a control the precipitate from lysates of bacteria transformed with the pATH vector alone were so dissolved and emulsified. The above emulsions were injected intradermally into rabbits (0.3-0.5 mg SmIMP25-TrpE fusion protein, or control, per injection) at 10 day intervals. Antiserum was collected 10 days after the fifth injection.

The antisera were used to immunoprecipitate cell-free translation products programmed by RNA from different developmental stages of schistosome (see Examples 2(d)(i) and (ii)). A 25.5KD protein, corresponding to the size of the protein predicted from the nucleotide sequence of the cDNA, was recognized by antisera to above the three fusion proteins. The 25.5KD protein was immunoprecipitated from translation products programmed by RNA of miracidia, sporocyst, cercaria and adult worm, but not of uninfected hepatopancreas, in agreement with the Northern blots showing mRNA at these developmental stages (Fig. 6, see Example 3). Controls of normal rabbit serum and of rabbit anti-vector (anti-TrpE), as well as antisera against fusion proteins of TrpE with schistosome HSP70 (HSP70-TrpE) and paramyosin (Para-TrpE) did not precipitate the 25.5KD protein, while an antisera against HSP70-TrpE and Para-TrpE precipitated the expected 70KD and 97KD proteins, respectively (not shown).

The specificity of the antibodies to SmIMP25 is further evidenced by mRNA-hybrid selection experiments, in which total RNA of adult worm was hybridized with immobilized cDNAs. Antibodies to the 134H-TrpE fusion protein precipitated a 25.5KD protein programmed by mRNA annealed with the 63HT cDNA and none from translation products programmed by mRNA annealed with HSP70 cDNA. Conversely, anti-HSP70-TrpE immunoprecipitated a 70KD protein programmed by mRNA annealed with HSP70 cDNA, and none from translation products programmed by mRNA annealed with 63HT cDNA. pBR322 DNA did not select any mRNA translatable into proteins reactive with the above antisera, as expected.

Western blots of schistosome protein lysates developed with antiserum to 134H-TrpE revealed a complex pattern of bands (not shown). Anti-134H-TrpE recognized in cercaria two proteins of about 40KD and 70KD, and in adult worm it reacted with a 40KD protein and faintly with a 25.5KD protein. The faint about 95KD band in cercaria and adult worm is not specific since it was also detected by normal rabbit serum (not shown), as well as by antisera to TrpE and to CaBP-TrpE (not shown). The 25.5KD protein corresponds in size to the 25.5KD protein precipitated from cell-free translation products and probably represents the unmodified protein backbone. The 40KD and 70KD proteins may represent extensively glycosylated forms of the same protein (the 25.5KD protein has three N-glycosylation sites, see Fig. 5) subjected to different degrees of modification in cercaria and adult worm. These bands were not detected by normal rabbit serum (data not shown), neither by rabbit antiserum against the pATH vector (not shown), nor by antiserum directed against the CaBP-TrpE encoding the cercarial specific calcium binding protein (Ram et al. 1989) which detected in *cercaria* the expected 8KD CaBP molecule (not shown).

It should also be noted that for the purposes of preparing the SmIMP25 protein of the invention, as noted above, vaccinia vectors, of vaccinia virus origin, may be used. For example, the above-noted cDNAs of SmIMP25 encoding 134 a.a., 175 a.a. and 222 a.a. may be inserted into an appropriate site in a suitably prepared vaccinia vector, using standard procedures, to provide effective expression of these cDNAs in a suitable host cell once said host cell is transformed by the so-prepared vaccinia vectors.

EXAMPLE 9:

Tissue homogenates and Western blots

Parasite tissue, snail hepatopancreas (HP) or mouse kidney were homogenized in 5-10 volumes of PBS containing protease inhibitors at final concentration of: 20 $\mu$l/ml $\alpha$-1-antitrypsin (Sigma), 0.1 $\mu$g/ml Soybean trypsin inhibitor (Sigma), 2 mM Tosylphenylalanine chloromethyl ketone (TPCK-Sigma), 2 mM Tosyllysine chloromethyl ketone (TLCK-Sigma), 2 mM Phenylmethanesulfonyl fluoride (PMSF-Sigma), Aprotonin (1:100, Sigma), and 10 mM EDTA (Levi-Schaffer et al. 1984). The homogenates were sonicated (50% duty cycle, output control 5) in a bath sonicator (Heat Systems Ultrasonics, Plainview, N.Y.) at 4°C for 3 min on ice, and vortexed (30 sec). Sonication and vortexing were repeated three times. The sonicates were kept at -70°C till used. Thawed sonicate (10 $\mu$g protein/lane) was separated on SDS-PAGE. Proteins were electro-blotted from SDS-PAGE to a nitro-cellulose filter in a 50 mM Tris-glycine buffer pH 8.3 for 3 hr at 190 mA at room temperature. To block non-specific binding, the filter was kept for 1 hr at room temperature in blocking solution (PBS containing 10% low fat milk), the filter was washed twice in PBS and then reacted with the antibody, prepared according to Example 8, (1:100 serum dilution) in PBS containing 20% fetal calf serum for 12-16 hr at 4°C. Filters were washed (x5) in the above blocking solution containing 0.05% Tween 20. A second antibody being an immuno-affinity purified goat anti-rabbit Ig labelled with $^{125}$I (~500,000 cpm/ml, ~50-100ng/ml) was added in the above blocking solution for 2 hr at room temperature. The filter was extensively washed as above, and exposed to X-ray film.

EXAMPLE 10:

Immunofluorescence assay of intact parasite

Live mechanically transformed schistosomula (Example 2(a)) and adult worms (Example 1) were washed once with PBS, then washed 3 times with blocking solution of DSM (Example 2(b)(i)) containing 0.5% BSA and 0.1% bovine globulin (Sigma, fraction II) to prevent non-specific binding. The parasites were concentrated by mild centrifugation and resuspended in 100 $\mu$l of rabbit antisera to SmIMP25-TrpE fusion proteins (Example 8), rabbit anti-TrpE or normal rabbit serum (1:2 dilution in above DSM blocking solution). Incubation with antisera was for 30 min at room temperature. The parasites were washed three times with DSM-blocking solution, suspended in 100 $\mu$l of fluorescein-conjugated goat anti-rabbit IgG (Bio-Yeda, Israel) diluted 1:20 in the above DSM-blocking solution, kept for 20 min at room temperature, washed again with DSM, and fixed in 3% paraformaldehyde for 1 hr on ice, followed by 1 hr at room temperature. The parasites were examined and photographed using a fluorescence microscope (Zeiss, Photomicroscope III) and a Kodak 400 ASA3X film.

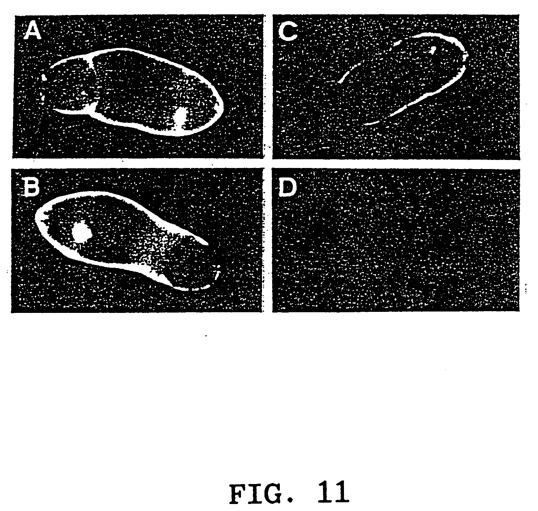

Integral membrane proteins may have been expected to have been located on intracellular membranes, on the surface of cells located inside the parasite, and/or on the surface tegument where host-parasite recognition/interaction takes place. The above immunofluorescence analyses of intact parasites have shown that the SmIMP25 protein is, in fact, exposed on the surface tegument of schistosomula and adult worm. Mechanically transformed schistosomula (Example 2(a)) were cultured in defined synthetic medium for 3 to 24 hr, and then reacted with rabbit antisera followed by staining with fluorescent anti-rabbit Fab. Strong surface fluorescence of 3 hr 24 hr schistosomula was obtained with antiserum against the 134H-TrpE (Fig. 11A, B, C, where A is antiserum reaction with 24 hr schistosomula and B and C are antiserum reactions with 3 hr schistosomula), and antiserum against 222H-TrpE (data not shown). Staining was not seen with several control sera: antiserum to TrpE (Fig. 11D), preimmune rabbit serum, rabbit antisera to fusion proteins in which TrpE was linked to schistosome HSP70, paramyosin or a calcium binding protein (data not shown). Adult worm showed surface fluorescence when reacted with anti-175-TrpE, but not when reacted with normal rabbit serum. Eggs were stained by rabbit anti-175-TrpE, but not by normal rabbit serum (data not shown).

EXAMPLE 11:

Immunogold electron microscopy

To gain information on the distribution of SmIMP25 inside the parasite immunogold electron microscopy was employed.

Cercariae and schistosomula (Example 2(a)) were fixed for 48 hr at 4°C in 3% paraformaldehyde, 0.1% glutaraldehyde, 0.1 M cacodylate buffer pH 7.4. Admut worms (Example 1) were fixed for 20 min at room temperature in the same fixative, cut into 3 mm pieces and immersed in the same fixative for 48 hr at 4°C. The parasites were then dehydrated in a series of graded alcohol, infiltrated with LR-gold (Bio-Rad) and polymerized under U.V. at room temperature. Ultrathin sections (0.1 $\mu$) were cut on LKB-Nova microtome. The sections were collected on nickel 200 mesh carbon coated paroldion grids. For labelling of sections with antibodies, grids were floated on droplets of the desired solutions. The blocking solution contained 1% Tween-20, 0.1% gelatine and 1% BSA in PBS. The first antiserum, prepared according to Example 8, being the various anti-SmIMP25-TrpE, was diluted 1:100 in the blocking solution and incubated with the grids for 2 hr at room temperature. The grids were washed 5 times for 5 min each in PBS and placed for 30 min on a drop of the second antiserum, being goat anti-rabbit Ig conjugated to 15 nm gold particles (Auroprobe EM, Gar G15, Janssen, Belgium) diluted 1:15 in PBS. This was followed by 3 washes in PBS and then distilled water. Finally the grids were stained with 2% uranyl acetate for 30 min in the dark. Electron micrographs were obtained using Philips EM140 electron microscope at 80kV.

The results of the above immunogold electron microscopy are summarized as follows: immunogold staining of cercariae, of 3 hr and 24 hr schistosomula and of adult worm showed SmIMP25 in several organs. Staining intensity differed with antisera to the various fusion proteins. Nevertheless, essentially the same results on SmIMP25 distribution were obtained with antisera to the three fusion proteins (see Example 8). For example, staining of preacetabular glands was stronger with anti-175-TrpE, yet it was evident, although at lower intensity, with anti-134H-TrpE and anti-222H-TrpE. On the other hand, staining of the surface tegument was strongest with anti-222H-TrpE, and less with anti-175-TrpE or anti-134H-TrpE. The variability in immunostaining intensity is not necessarily due to immunization by different constructs of

16

SmIMP25-TrpE. It can be attributed, at least in part, to differences in the polyclonal antibody responses of different rabbits. This may be so because the antisera of two rabbits immunized with the same 175-TrpE fusion protein revealed comparable variability in tissue staining.

Acetabular glands. Both pre- and post-acetabular glands were immuno-stained with the various anti-SmIMP25-TrpE. Staining was stronger in pre-acetabular glands compared to post-acetabular glands. In pre-acetabular glands staining was confined to the homogeneous electron dense type B secretory globules, and staining was stronger in cercariae than in 3 hr schistosomula.

Head gland. Immuno-staining for SmIMP25 was strong in secretory granules of cercariae and much less in 3 hr schistosomula.

Subtegumental cells. The subtegumental cells are involved in the synthesis of the syncytial tegument that covers the surface of the *schistosome*. The tegumental nuclei are located not within the surface syncytium but within sunken subtegumental cells situated beneath the muscle layer. The cell bodies are irregular in shape, sometimes multinucleate, containing inclusion bodies, free ribosomes, rough endoplasmic reticulum, Golgi and few mitochondria. The subtegumental cells are joined to the tegument by one or more long, tortuous cytoplasmatic connections, containing microtubules. Continuity between the cells and the surface tegument is rarely seem in sectioned material. These cells may be responsible for the synthesis of the tegument in cercariae, schistosomula and adult worms, and the inclusion bodies of the subtegumental cells may be translocated into the tegument via the microtubule-lined cytoplasmatic connections. Globules containing SmIMP25 were found in subtegumental cells of cercariae and in subtegumental cells of adult worm. In addition, adult worm had globules of variable size, shape and electron density-, heavily stained with anti-SmIMP25. These globules were dispersed in the parenchyma or in the cytoplasma of parenchymal cells. Apparently, SmIMP25 synthesized in the cell is packaged into globules that are released to the parenchyma. This may indicate that these globules migrate to the surface and are incorporated into the tegument.

Surface tegument. The surface tegument is a syncytium connected by cytoplasmic bridges to subtegumental cells sunken in the parenchyma. The outer plasma membrane of the tegument of cercariae is trilaminated. During cercaria-schistosomula transformation it duplicates into a heptalaminated bilayer maintained in schistosomula, lung worms and adult worms. The cercaria is covered by a thick coat called glycocalyx. The glycocalyx is rapidly lost during transformation to schistosomula. The glycocalyx is greatly reduced in 1 hr schistosomula, but remnants persist for several hours, mainly in mechanically transformed schistosomula. During cercaria-schistosomula transformation the tegument is subjected to extensive modifications required for adaptation to parasitic life in the vertebrate host. In adult worm the tegument shows rapid turnover, mainly of the outer bilayer, resulting in the release of surface antigens to the surroundings. The surface tegument of cercaria, as well as of 3 hr and 24 hr schistosomula was heavily stained for SmIMP25. Staining was mainly located on the outer layer of the tegument, and in amorphous material adjacent to the surface that resembles remnants of the glycocalyx. The spines were also stained but at lower intensity. The lucid interstitial layer below the tegument is notably free of SmIMP25. The specificity of staining is also evidenced by the lack of staining in tegument reacted with normal rabbit serum. Immuno-staining of adult worm tegument was weaker compared to the invasive larvae, yet SmIMP25 was detected inside the tegument and spines of the adult worms.

Different patterns of tegument staining were obtained when antisera to SmIMP25 and to CaBP were reacted with 3 hr schistosomula. As mentioned above, the SmIMP25 was mainly confined to the outer surface. On the other hand, the CaBP was distributed throughout the thickness of the tegument, and it was not concentrated on the outer surface. Accordingly, immunofluorescent staining of intact schistosomula was obtained with anti-SmIMP25-TrpE (Fig. 11) but not with anti-CaBP-TrpE (data not shown).

Flame cells. The excretory unit of the parasite is the unicellular flame cell embedded in the parenchyma. The flame cell has a basal cytoplasmatic portion which contains the nucleus and bears a characteristic bundle of cilia extending into the lumen of the collecting tubules. In adult worm the basal cytoplasmatic portion of the flame cell and the cytoplasma of the excretory tubule cell showed significant immuno-staining for SmIMP25, while the bundle of cilia showed very little staining. The cilia were practically devoid of SmIMP25 similar to adult worm. On the other hand, the cilia of cercariae were heavily stained for the CaBP. There is no significant staining for SmIMP25 in the lumen of the flame cells, that is, it seems that SmIMP25 is not excreted by the flame cell system.

EXAMPLE 12:

Immunological properties of SmIMP25

Surface antigens have been studied extensively as preferred targets for protective immune responses. It was therefore of interest to study the immune potential of the cloned SmIMP25 (Examples 8, 9) which is an integral membrane protein exposed on the surface of the parasite, and compare it with three non-surface antigens of *schistosome* previously cloned: paramyosin (Para), a calcium binding protein (CaBP), and heat shock protein 70 (HSP70). All the antigens were prepared as TrpE fusion proteins in the pATH expression vector. The 134H-TrpE, 175-TrpE and 222H-TrpE, are derived from SmIMP25 as described above in Example 9 (Fig. 8). The Para-TrpE recombinant encodes about half (52 KD) of the paramyosin molecule, and antibodies to this fusion protein recognize the intact protein (97 KD) in parasite lysates (Grossman et al, 1990). The CaBP-TrpE recombinant encodes the complete CaBP molecule (8 KD) (Ram et al., 1989), and antibodies to this fusion protein recognize the intact molecule (8 KD) in parasite lysates. The HSP70-TrpE recombinant encodes 315 a.a. of the C-terminus of the HSP70, and antibodies to this fusion protein recognize the intact protein (70 KD) in the parasite lysates. Rabbit antibodies to the latter antigens did not stain the surface of schistosomula demonstrating that the Para, CaBP and HSP70 are not exposed on the surface of the parasite (data not shown). The antisera against the *schistosome* fusion proteins were analyzed as follows:

a) *In−vitro* complement dependent killing of schistosomula. One hundred 3 hr schistosomula were suspended in separate tubes of 150 $\mu$l DSM each containing rabbit antibodies to one of the various above SmIMP25-TrpE fusion proteins, prepared according to Examples 8, 9 (1:4 dilution) and guinea pig complement (1:10 dilution of fresh guinea pig serum), and incubated at 37°C for 18 hr. Same samples but with heat inactivated complement served as one control and antisera to TrpE and normal rabbit sera (NRS) served as other controls. The percent of dead schistosomula was determined by microscope examination.

Antisera to 134H-TrpE showed highest level of toxicity (75% killing) similar (actually somewhat higher) to the level obtained with rabbit antisera raised against whole cercarial extract (68%). So far anti-cercarial extract caused maximal killing in this assay, therefore it is likely that anti-134H-TrpE is very toxic to the parasite. Antisera to CaBP-TrpE, Para-TrpE and HSP70-TrpE showed 40%, 35% and 29% killing of schistosomula, respectively. Killing activity was due to antibodies against the moiety of the parasite protein in the fusion protein since antisera to TrpE showed very low toxicity (9% killing) similar to normal rabbit sera (6% killing). Thus, using an *in−vitro* assay, antisera to the surface antigen were more toxic than antisera to non-surface antigens (Table 2).

## TABLE 2

**Susceptibility of schistosomula to antibodies and complement**
Results obtained with the antisera of two rabbits (enclosed in brackets) and the average, are given. Numbers refer to the percent of dead schistosomula in the presence of antibodies and complement corrected for the control of dead schistosomula in the presence of antibodies and heat-inactivated complement.

| Specificity of antibodies | Percent of killing | |
|---|---|---|
| | average | (two individual rabbits) |
| 134H-TrpE | 75 | (76, 74) |
| CaBP-TrpE | 40 | (57, 24) |
| Para-TrpE | 35 | (42, 28) |
| HSP70-TrpE | 29 | (42, 16) |
| TrpE | 9 | (11, 7) |
| NRS | 6 | (8, 4) |
| Cercarial extract | 68 | (70, 66) |

b) Protection of mice against challenge infection. The various SmIMP25-TrpE fusion proteins, noted above, TrpE and the various above non-surface antigens synthesized in bacteria served as antigens. Bacterial lysates were centrifuged, precipitates enriched with the antigens were dissolved in PBS supplemented with SDS (1%) and Triton-X-100 (1%), and used to immunize groups of C57Bl/6J mice aged 2 month. The antigen (10 $\mu$g/injection) was incorporated in complete Freund's adjuvant and three injections were administered at 2 week intervals: two into the footpad and the 3$^{rd}$ injection subcutaneously. One group received only one injection into the footpad. Thirteen days after the last injection the mice were challenged with 90 *S.mansoni* cercariae per mouse by belly skin penetration. Seven weeks later liver perfusion was performed and recovered worms were counted.

The results are presented in Table 3, which illustrate that immunization with the SmIMP25 fusion proteins consistently gave significant levels of protection. The 134H-TrpE construct conferred 33% and 32% protection in two groups of mice receiving three injections of the antigen, and 30% protection in a third group of mice immunized by a single injection. A similar level of protection (34%) was obtained by immunization with 222H-TrpE. The 175-TrpE was the best antigen, eliciting the highest level of protection (46%) against challenge infection.

TABLE 3

Protection of mice against challenge infection by immunization with surface and non-surface antigens. Three injections of the antigen (10μg/injection) were administered in complete Freund's adjuvant at 2 week intervals. 13 days after the last injection the mice were challenged with 90 cercariae per mouse and the level of protection was assayed seven weeks later by liver perfusion. Asterisk indicates one group of mice receiving a single injection of the 134H-TrpE antigen (10μg/injection) followed by cercarial infection and liver perfusion as above.

| Immunizing antigen | Experiment 1 | | | Experiment 2 | | | Experiment 3 | | |
|---|---|---|---|---|---|---|---|---|---|
| | Mice In group | Worms/mouse (average±SD) | Protection (%) | Mice In group | Worms/mouse (average±SD) | Protection (%) | Mice In group | Worms/mouse (average±SD) | Protection (%) |
| None | 8 | 36.9±3.6 | | 9 | 42.3±3.7 | | 5 | 42.0±4.2 | |
| TrpE | 7 | 33.3±3.2 | 10 | 9 | 42.5±3.4 | - | 8 | 36.8±8.3 | 12 |
| 134H-TrpE | | | | 11 | 28.5±3.4 | 33 | 10 | 28.5±3.5 | 32 |
| 175-TrpE | | | | 9* | 29.8±3.5 | 30 | 8 | 22.5±7.1 | 46 |
| 222H-TrpE | | | | | | | 9 | 27.6±4.0 | 34 |
| Para-TrpE | 8 | 24.4±4.2 | 34 | | | | 8 | 33.7±3.3 | 20 |
| CaBP-TrpE | 6 | 26.3±1.9 | 28 | | | | 10 | 30.5±9.2 | 27 |
| HSP70-TrpE | 9 | 27.9±2.9 | 24 | | | | 9 | 34.6±5.8 | 18 |

Protection levels achieved with the non-surface antigens tested were lower compared to the SmIMP surface antigen. Each antigen was tested in two groups of mice. The CaBP-TrpE conferred 28% and 27% protection (average 28%), and HSP70-TrpE conferred 24% and 18% protection (average 21%). Para-TrpE gave 34% and 20% protection in two experiments, i.e. the results obtained with this antigen were more variable than the results obtained with CaBP-TrpE, HSP70-TrpE and 134H-TrpE that were fairly reproducible. Such variability is not unexpected considering the infection of mice with cercariae and the recovery of adult worm by liver perfusion. These results with Para-TrpE immunization (34%, 20%, average 27% protection) are similar to previously reported data on another recombinant of paramyosin that conferred 30% and 22% (average 26%) protection against challenge infection in mice (Pearce et al., 1988). Immunization of three groups of mice with lysates enriched with the TrpE protein, serving as control, gave borderline levels of protection, if any (10%, 0, 12%, average 7% protection).

Thus, in view of the aforementioned in (a) and (b) above, it may be concluded that *In−vitro* complement-dependent toxicity assays against schistosomula showed that antisera to SmIMP25 (75% killing) were more toxic than antisera to CaBP (40% killing), paramyosin (35% killing) or HSP70 (29% killing). The toxicity of antisera to SmIMP25 (75% killing) may represent the maximal toxic effect attainable in this assay, since it was comparable (actually somewhat higher) to the toxicity of antisera to whole cercarial extract (68% killing) (Table 2). Immunization of mice with different constructs of SmIMP25 conferred 30-46% protection against challenge infection with *S.mansoni*. The highest level of protection (46%) was achieved with the 175-TrpE construct encoding 175 amino acids exposed on the outer surface of the parasite, but without the transmembrane and cytoplasmic domains (see Fig. 8). Protection induced by immunization with the non-surface antigens was lower: 27% protection with CaBP or paramyosin, and 21% protection with HSP70 (Table 3).

The 46% protection induced by immunization with the 175-TrpE construct of SmIMP25 is at the highest level of protection reported for other antigens in the mouse model of infection. Vaccination with live radiation-attenuated cercariae induced 43% protection (McLaren and Smithers, 1985). Whole worm homogenate and adult worm surface membranes injected with saponin provoked 18-36% (average 27%) protection (Smithers et al., 1989). The 9B antigen preparation induced 34% protection (Tarrab-Hazdai et al., 1985). Native paramyosin purified from *S.mansoni* induced 39% protection, and the recombinant paramyosin induced 26% protection (Pearce, et al., 1988). Immunization of mice with the 28 KD GST purified from *S.mansoni* conferred 42% protection against challenge infection (Balloul et al., 1987).

The World Health Organization long argued that Bilharzia can be controlled by drugs and sanitation alone. Praziquantel, the drug of choice, kills adult worms and cures the infection. However, people in the endemic areas, mainly children, become reinfected from larvae in water snails. Since 1984 Bilharzia has spread to two more countries - 76 in all. Therefore WHO officials have recently announced that "Schistosomiasis vaccine development has a very high priority. It is a big hole in our repertory". The vaccine need not provide >90% protection as in viral or bacterial diseases because the parasite does not reproduce within the human host, and debilitating symptoms of the disease generally appear in people with a high number of parasites. Thus, a vaccine reducing the number of worms by 50% or more, can dramatically reduce the number of severe cases of the disease.

## REFERENCES

Balloul, J.M., Sondermeyer, P., Dreyer, D., Capron, M., Grzych, J.M., Pearce, R.J., Carvallo, Dr., Lecocq, J.P. and Capron, A. (1987). Molecular cloning of a protective antigen of *schistosome*. Nature 326: 149-153.

Dalton, J.P., Tom, T.D. and Strand, M. (1987). Cloning of a cDNA encoding a surface antigen of *Schistosoma mansoni* schistosomula recognized by sera of vaccinated mice. P.N.A.S. 84: 4268-4272.

Grossman, Z., Ram, D., Markovics, A., Tarrab-Hazdai, R., Lantner, F., Ziv, E. and Schechter, I. (1990). *Schistosoma mansoni*: stage specific expression of muscle specific genes. Exp. Parasitology. 70: 62-71.

Harn, D.A., Mitsuyama, M., Huguenel, E.D., and David, J.R. (1985). *Schiztosoma mansoni*: Detection by monoclonal antibody of a 22.000 dalton surface membrane antigen which may be blocked by host molecules on lung stage parasites. J. Immunol. 135: 2115-2120.

Havercroft, J.C., Huggins, M.C., Neve, V., Dunne, D.W., Richardson, B.A., Taylor, D.W. and Butterworth, A.E. (1988). Cloning of the gene encoding 50 kilodalton potential surface antigen of *Schistosoma mansoni*. Mol. Biochem. Barasitol. 30: 83-88.

Jeffs, S.A. et al., (1991). Molecular cloning and characterisation of the 22-kilodalton adult *Schistosoma mansoni* antigen recognised by antibodies from mice protectively vaccinated with isolated tegumental surface membranes. Mol. and Biochem. Parasitol. 46: 159-168.

Knight, M., Kelly, C., Rodrigues, V., Yi, X., Wamachi, A., Smithers, S.R. and Simpson, A.J.G. (1989). A cDNA clone encoding part of the major 25,000-dalton surface membrane antigen of adult *Schistosoma mansoni*. Parasitol. Res. 75: 280-286.

Lanar, E.D., Pearce, E.J., James, S.L. and Sher, A. (1986). Identification of paramyosin as schistosome antigen recognized by intradermally vaccinated mice. Science 234: 593-596.

Levi-Schaffer, F., Schryer, M.D. and Smolarsky, M. (1982). The resistance of schistosomula of *Schistosoma*

mansoni to antibodies and complement in – vitro does not correlate with the binding of antibodies to the surface fo the parasite. J. Immunol. 129: 2744-2751.

Levi-Schaffer, F., Tarrab-Hazdai, R., Schryer, M.D., Arnon, R. and Smolarsky, M. (1984). Isolation and partial characterization of the tegumental outer membrane of schistosomula of Schistosoma mansoni. Mol. Biochem. Parasitol. 13: 283-300.

Maniatis, T., Fritsch, E.F. and Sambrook, J. (1989, 2nd edition). Molecular Cloning, A Laboratory Manual; Cold Spring Harbor Laboratory Press, Cold Spring Harbor, New York; pages A1-A7; 2.73-2.76.

McLaren, D.J. and Smithers, S.R. (1985). Schistosoma mansoni. Challgene attrition during the lung phageof migration in vaccinated and serum protected rats. Exp. Parasitol. 60:1-9.

Pearce, E.J., James, S.L., Hieny, D.E., Lanar, D.E. and Sher, A. (1988). Induction of protective immunity against Schistosoma mansoni by vaccination with schistosome paramyosin (Sm97), a non surface parasite antigen. Proc. Natl. Acad. Sci. USA 85: 5678-5682.

Ram, D., Grossman, Z., Markovics, A., Avivi, A., Ziv, E. Zantner, F. and Schechter, I. (1989). Rapid changes in the expression of a gene encoding a calcium binding protein of Schistosoma mansoni. Mol. Biochem. Parasitology 34: 167-176.

Smith and Clegg, (1985). Vaccination against Schistosoma mansoni with purified surface antigens. Science. 227: 535-538.

Smith, D.B., Rubira, M.R., Simpson, R.J. Davern, K.M. Tlu, W.U.., Board, P.G. and Mitchell, G.F. (1988). Expression of an enzymatically active parasite molecule in Escherichia coli: Schistosoma japonicum glutathione S.transferase. Mol. Biochem. Parasitol. 27: 249-256.

Smithers, S.R., Hackett, F., Omer-Ali, P. and Simpson, A.J.G. (1989). Protective immunization of mice against Schistosoma mansoni with purified adult worm surface membranes. Parasite Immunol. 11: 301-318.

Stein, L.d. and David, J.R. (1986). Cloning of a developmentally regulated tegument antigen of Schistosoma mansoni. Mol. Biochem. Parasitol. 20: 253-264.

Tanase, N., Roth, M.J. and Goff, S.P. (1986). Analysis of retroviral pol gene products with antisera raised against fusion proteins produced in Escherictia coli. J. Virology. 59: 328-340.

Tarrab-Hazdai, R., Levi-Schaffer, F., Brenner, V., Horowitz, S., Eshhar, Z. and Arnon, R. (1985). Protective monoclonal antibody against Schistosoma mansoni: antigen isolation, characterization and suitability for active immunization. J. Immunol. 135: 2772-2779.

Taylor, J.B., Vidal, A., Torpier, G., Meyer, D.J., Roitsch, C., Balloul, J.M., Southan, C., Somdermeyer, P., Pemble, S., Lecocq, J.P., Capron, A. and Ketterer, B. (1988). The glutathione transferase activity and tissue distribution of a cloned $M_r$28K protective antigen of Schistosoma mansoni. EMBO J. 7: 465-472.

Trottein, F., Kieny, M.P., Verwaerde, C., Torpier, G., Pierce, R.J., Balloul, J.M., Schmitt, D., Lecocq, J.P. and Capron, A. (1990). Molecular cloning and tissue distribution of a 26-kilodalton Schistosoma mansoni glutathione S-transferase. Mol. Biochem. Parasitol. 41: 35-44.

Wright, M.D., Henkle, K.J. and Mitchell, G.F. (1990). An immunogenic $M_r$ 23,000 integral membrane protein of Schistosoma mansoni worms that closely resemble a human tumor-associated antigen. J. Immunol. 144: 3195-3200.

Wright, M.D., Davern, K.M. and Mitchell, G.F. (1991). The functional and immunological significance of some schistosome surface molecules. Parasitol. Today 7: 56-58.

SEQUENCE LISTING


(1) GENERAL INFORMATION:

    (i) APPLICANT:
        (A) NAME: Yeda Research And Development Company Limited
        (B) STREET: P.O. Box 95
        (C) CITY: Rehovot
        (E) COUNTRY: Israel
        (F) POSTAL CODE: 76100

    (ii) TITLE OF INVENTION: Vaccine Against Schistosomiasis

    (iii) NUMBER OF SEQUENCES: 8

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (v) CURRENT APPLICATION DATA:
        (A) APPLICATION NUMBER: 93300573.8
        (B) FILING DATE: 27-JAN-1993


(2) INFORMATION FOR SEQ ID NO:1:

    (i) SEQUENCE CHARACTERISTICS:
        (A) LENGTH: 36 base pairs
        (B) TYPE: nucleic acid
        (C) STRANDEDNESS: single
        (D) TOPOLOGY: linear


    (xi) SEQUENCE DESCRIPTION: SEQ ID NO:1:

CCACTTTTAG CCCTTTGAAA TCTTGTACCA TCACTG            36

(2) INFORMATION FOR SEQ ID NO:2:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear


    (xi) SEQUENCE DESCRIPTION: SEQ ID NO:2:

GTACCATCAC TGAGAAAATT            20

(2) INFORMATION FOR SEQ ID NO:3:

    (i) SEQUENCE CHARACTERISTICS:
        (A) LENGTH: 16 base pairs
        (B) TYPE: nucleic acid
        (C) STRANDEDNESS: single
        (D) TOPOLOGY: linear

23

(xi) SEQUENCE DESCRIPTION: SEQ ID NO:3:

TCATTTTCAC TCTAGT                                                    16

(2) INFORMATION FOR SEQ ID NO:4:

    (i) SEQUENCE CHARACTERISTICS:
        (A) LENGTH: 16 base pairs
        (B) TYPE: nucleic acid
        (C) STRANDEDNESS: single
        (D) TOPOLOGY: linear


(xi) SEQUENCE DESCRIPTION: SEQ ID NO:4:

CACTTTATTC TTCAGG                                                    16

(2) INFORMATION FOR SEQ ID NO:5:

    (i) SEQUENCE CHARACTERISTICS:
        (A) LENGTH: 16 base pairs
        (B) TYPE: nucleic acid
        (C) STRANDEDNESS: single
        (D) TOPOLOGY: linear


(xi) SEQUENCE DESCRIPTION: SEQ ID NO:5:

TTTACTGTAT TTCAGG                                                    16

(2) INFORMATION FOR SEQ ID NO:6:

    (i) SEQUENCE CHARACTERISTICS:
        (A) LENGTH: 16 base pairs
        (B) TYPE: nucleic acid
        (C) STRANDEDNESS: single
        (D) TOPOLOGY: linear


(xi) SEQUENCE DESCRIPTION: SEQ ID NO:6:

AACGTTTTTC TTCAGG                                                    16

(2) INFORMATION FOR SEQ ID NO:7:

    (i) SEQUENCE CHARACTERISTICS:
        (A) LENGTH: 1759 base pairs
        (B) TYPE: nucleic acid
        (C) STRANDEDNESS: double
        (D) TOPOLOGY: linear


    (ix) FEATURE:
        (A) NAME/KEY: CDS
        (B) LOCATION: 51..722

24

(xi) SEQUENCE DESCRIPTION: SEQ ID NO:7:

```
AGATAGGTGC ACAGTTTAAA ATGCTGGTCC GATTGTCTGT TTTCTGAACG ATG GAT          56
                                                        Met Asp
                                                          1

CTC CGC TAC TTT GCT ATT ACA AAC GAG AAA CAT ATT GTT TGG TAT CGG        104
Leu Arg Tyr Phe Ala Ile Thr Asn Glu Lys His Ile Val Trp Tyr Arg
          5                   10                  15

TCG TTC ACA AAA CGT AGT TTT GAA GAT GTA GTA CTT AAA TAT CTG TTA        152
Ser Phe Thr Lys Arg Ser Phe Glu Asp Val Val Leu Lys Tyr Leu Leu
        20                  25                  30

TGT AAT CCT CCA GAT CAA GAT ATA TAT TAT AGC GAA GGC GAT TTC TCT        200
Cys Asn Pro Pro Asp Gln Asp Ile Tyr Tyr Ser Glu Gly Asp Phe Ser
 35                  40                  45                  50

CTT CAC TGC TTA ACC GTT TCA GGA CTT TCC TTT ATT GCG GTA ACC GAT        248
Leu His Cys Leu Thr Val Ser Gly Leu Ser Phe Ile Ala Val Thr Asp
                55                  60                  65

CGA AAC GCA TCT CGA CAA AAA TTT CGA AAT TTG GTT GTG GAG GTT TCT        296
Arg Asn Ala Ser Arg Gln Lys Phe Arg Asn Leu Val Val Glu Val Ser
                70                  75                  80

CGT AAT TTT CTC AGT GAT GGT ACA AGA TTT CAA AGG GCT AAA AGT GGT        344
Arg Asn Phe Leu Ser Asp Gly Thr Arg Phe Gln Arg Ala Lys Ser Gly
            85                  90                  95

GGT AAA GAT TGT TTA CAA CAG TCT TAT AGT CTG AAC TTG GAA GAG TTG        392
Gly Lys Asp Cys Leu Gln Gln Ser Tyr Ser Leu Asn Leu Glu Glu Leu
        100                 105                 110

ATG GAA TCA CCC AAC TCT TCT AAG GGA AAT GGT TCG TTG GAC CAG TTA        440
Met Glu Ser Pro Asn Ser Ser Lys Gly Asn Gly Ser Leu Asp Gln Leu
115                 120                 125                 130

CGT AGT AAT GTG AAT ACT GTA ACA GCT GTA ATG CAA GAG AAT ACT AGA        488
Arg Ser Asn Val Asn Thr Val Thr Ala Val Met Gln Glu Asn Thr Arg
                135                 140                 145

CTA GCG CTT CAA CGA GGT GAT CGG TTA AAC AAT TTG GTC AGT AAA ACA        536
Leu Ala Leu Gln Arg Gly Asp Arg Leu Asn Asn Leu Val Ser Lys Thr
                150                 155                 160

GAT GAA CTT GCA GCC AGC GCT AAT CAA TTC CAA ACA ACA GCC ACA AAG        584
Asp Glu Leu Ala Ala Ser Ala Asn Gln Phe Gln Thr Thr Ala Thr Lys
            165                 170                 175

GTG GCC CGA AAA ACT TGG TGG GAA AAT TTC CGT ATG AAA TTA ATT ATT        632
Val Ala Arg Lys Thr Trp Trp Glu Asn Phe Arg Met Lys Leu Ile Ile
        180                 185                 190

GTT GGT GTT GTC GGT GGC ATA CTT CTA GTA ATT GTG ATA ATT ATA CTC        680
Val Gly Val Val Gly Gly Ile Leu Leu Val Ile Val Ile Ile Ile Leu
195                 200                 205                 210

TGG CAG ACT GGG GTG TTC AAT AGC AAA TCG CAA CAC CAG TAATAATAGC         729
Trp Gln Thr Gly Val Phe Asn Ser Lys Ser Gln His Gln
                215                 220

AACAGTAAAT AATTAACTTC ATATTGTTTA CTACATGAAA CAGAAAATGC AAAGGAACAC       789

CCATCTGAAA ATTAGGTGAG ATATTCGATA ATGTGTTTTT TTTACTGGAT CAATTTATAC       849

AACACGTCTT GTTAACATTT CAATTTTCTT TATCGCGACA CGTTCTCCTT TTGTTTCCCT       909
```

```
CGTGACAGAT AAATTTTACA AATGATGATG ACAATGATTA ATATTGCACA GTATATTGTA    969

ATTTTTATGC TATATTATAA ATATACATAC TTAAGTCTTT TTTTGTAATC ACTATACCTA   1029

CTACTAATCC TGCATTAACT ACTTCTCACT GATGAAATAT CTTTTCTATA TAGGCGCAGA   1089

GATTTAATGA CAATTACATA TCATATATAT ATATATATAT ATATATATAT ATATAGTGTT   1149

CGGAATGATT TTTTCTCTTA AACGAATTTA TTTTTCACTC AACATCTATC TACTTTGTAT   1209

GGGTGACTAT CTTCCCAATG TATATCGATT ATGTGTTCTT TATTGATCCC AATACACATT   1269

TTGTTTATTT TCAATAACAA TGACTTTTTG AAAGCCAAAG ATTATCAATA AATGAAAGAA   1329

GATAAAAGAG GTACAAACTG TTTGATATCT TTTTGTTGGA ATTACACTTT GTAAACGCAT   1389

GCATGTGTCT TTATGCGTGC GTGTGTGCAT ATATGTTCAA GTGGCTTCAT AATAATACCA   1449

CAACTATTCA CTTTTATCAT TATTTCCGTG CCCTGTCCTT TTTACTTCTC TACAGATAAT   1509

TTAAAGTATA CTCATTTCTG TACTATTTCT TCTTGCGGTT ATTCTCATTA CTATTAACAT   1569

TATGAGAAAA TGTGTATTTG CAATTTCTAC TCTGGAAGAA TTCCTTCTTA TTATTGATTA   1629

GTACTTATCA TGGTTTTACT TTTTACCAGA TTTAAATTAA TATGTTTGCT CAATATACAT   1689

AAATTTTCTT TTCCTTTTTT TTAACAAAAA TCATATTTGT CATGTATTGA ATACCAACAA   1749

AATTGAATTC                                                          1759
```

(2) INFORMATION FOR SEQ ID NO:8:

      (i) SEQUENCE CHARACTERISTICS:
          (A) LENGTH: 223 amino acids
          (B) TYPE: amino acid
          (D) TOPOLOGY: linear

      (ii) MOLECULE TYPE: protein

      (xi) SEQUENCE DESCRIPTION: SEQ ID NO:8:

```
Met Asp Leu Arg Tyr Phe Ala Ile Thr Asn Glu Lys His Ile Val Trp
 1               5                  10                  15

Tyr Arg Ser Phe Thr Lys Arg Ser Phe Glu Asp Val Val Leu Lys Tyr
            20                  25                  30

Leu Leu Cys Asn Pro Pro Asp Gln Asp Ile Tyr Tyr Ser Glu Gly Asp
        35                  40                  45

Phe Ser Leu His Cys Leu Thr Val Ser Gly Leu Ser Phe Ile Ala Val
    50                  55                  60

Thr Asp Arg Asn Ala Ser Arg Gln Lys Phe Arg Asn Leu Val Val Glu
65                  70                  75                  80

Val Ser Arg Asn Phe Leu Ser Asp Gly Thr Arg Phe Gln Arg Ala Lys
                85                  90                  95

Ser Gly Gly Lys Asp Cys Leu Gln Gln Ser Tyr Ser Leu Asn Leu Glu
            100                 105                 110

Glu Leu Met Glu Ser Pro Asn Ser Ser Lys Gly Asn Gly Ser Leu Asp
            115                 120                 125
```

26

EP 0 554 064 A1

```
Gln Leu Arg Ser Asn Val Asn Thr Val Thr Ala Val Met Gln Glu Asn
    130             135         140

Thr Arg Leu Ala Leu Gln Arg Gly Asp Arg Leu Asn Asn Leu Val Ser
145             150         155             160

Lys Thr Asp Glu Leu Ala Ala Ser Ala Asn Gln Phe Gln Thr Thr Ala
            165         170         175

Thr Lys Val Ala Arg Lys Thr Trp Trp Glu Asn Phe Arg Met Lys Leu
            180         185         190

Ile Ile Val Gly Val Val Gly Gly Ile Leu Leu Val Ile Val Ile Ile
        195         200         205

Ile Leu Trp Gln Thr Gly Val Phe Asn Ser Lys Ser Gln His Gln
    210         215         220
```

## Claims

1.  An immunogenically and antigenically active protein capable of eliciting immunity against *Schistosoma* parasites, selected from the group consisting of:

    (a) *Schistosoma mansoni* integral membrane protein having in the unglycosylated form a molecular weight of about 25 KD (SmIMP25);

    (b) analogs of (a) obtained by deletion, addition or replacement of one or more amino acids or obtained by glycosylation at one or more glycosylation sites of (a) without substantially affecting the immunogenicity of said protein;

    (c) fragments of (a) or (b) comprising a portion of or all of the extracellular region of said protein or comprising a portion of or all of the intracellular and transmembrane region and a portion of the extracellular region of said protein, wherein said fragments substantially retain the antigenicity and immunogenicity of said protein; and

    (d) fusion polypeptides comprising any one of (a), (b), or (c) fused to a suitable bacterial, phage or viral protein.

2.  The protein according to claim 1, selected from the group consisting of:

    (i) SmIMP25 having the amino acid sequence depicted in Fig. 5;

    (ii) SmIMP25 in glycosylated form, being glycosylated at one or more of the glycosylation sites at positions 68-70, 119-121 and 124-126 of the sequence depicted in Fig. 5;

    (iii) the analog designated herein as 222H having the amino acid sequence depicted in Fig. 5 without the N-terminal methionine residue at position 1 of said sequence;

    (iv) the analog 222H of (iii) in glycosylated form, being glycosylated at one or more of the glycosylation sites at positions 68-70, 119-121 and 124-126 of the sequence depicted in Fig. 5;

    (v) the fragment designated herein as 134H having the amino acid sequence depicted in Fig. 5 from position 90 to position 223 of said sequence, wherein the amino acids from about position 192 to 223 comprise the transmembrane and intracellular regions of said protein;

    (vi) the fragment 134H of (v) in glycosylated form, being glycosylated at either or both of the glycosylation sites at positions 119-121 and 124-126 of the sequence depicted in Fig. 5;

    (vii) the fragment depicted herein as 175 having the amino acid sequence depicted in Fig. 5 from position 2 to position 176 of said sequence, said 175 amino acids being entirely in the extracellular region of said protein; and

    (viii) the fragment 175 of (vii) in glycosylated form, being glycosylated at one or more of the glycosylation sites at positions 68-70, 119-121 and 124-126 of the sequence depicted in Fig. 5.

3.  A fusion polypeptide according to claim 1, in which said bacterial, phage or viral protein is the bacterial TrpE protein encoded by the TrpE gene of the pATH expression vector, as herein described, which is fused to a protein, analog or fragment according to claim 2, said fusion polypeptide being SmIMP25-TrpE, 222H-TrpE, 175-TrpE or 134H-TrpE, as designated herein.

27

4. A vaccine comprising at least one of the proteins, analogs, fragments or fusion polypeptides according to any one of claims 1-3 and a pharmaceutically acceptable excipient or carrier for administration into humans or animals, said vaccine capable of eliciting in humans or animals an immune response that affords protection against infection by *Schistosoma* parasites.

5. A vaccine according to claim 4 said vaccine being a composite vaccine comprising two or more *Schistosoma* antigens, at least one of said antigens being selected from said proteins, analogs, fragments or fusion polypeptides of any one of claims 1-3.

6. Antibodies raised against any one of the proteins, analogs, fragments or fusion polypeptides according to any one of claims 1-3, said antibodies capable of binding to *Schistosoma* parasites.

7. A diagnostic method for the detection of the presence of *Schistosoma* antigens in subjects, comprising contacting antibodies according to Claim 6 with a test sample obtained from one or more of said subjects' body fluids, body excretions or organ tissues, and determining binding of said antibodies to antigens in said test sample.

8. An isolated DNA molecule encoding the proteins, analogs, fragments or fusion polypeptides according to any one of claim 1, claim 2 (i), (iii), (v), (vii), and claim 3.

9. A DNA molecule according to claim 8 comprising all or part of the sequence depicted in Fig. 4, encoding the SmIMP25 protein, the 222H analog and the 134H and 175 fragments of said SmIMP25 protein.

10. A recombinant DNA vector comprising the DNA molecule of claim 8 or claim 9 and control sequences for its propagation, maintenance and expression in prokaryotic host cells and/or its propagation, maintenance and expression in eukaryotic host cells.

11. A recombinant DNA vector according to claim 10, in which said DNA sequence of claim 8 is inserted in correct reading frame with a suitable bacterial, phage or viral protein to obtain a fusion polypeptide product according to claim 1 upon expression of said vector in a suitable host.

12. A recombinant DNA vector according to claim 10 being the bacterial pATH vector wherein said sequence of claim 9 is within the TrpE gene of said vector to obtain a fusion polypeptide according to claim 3 upon expression of the vector.

13. A suitable microorganism host cell transformed by the vector according to any one of claims 10-12.

14. A method for the preparation of the proteins, analogs, fragments and fusion polypeptides according to any one of claim 1, claim 2 (i), (iii), (v), (vii), and claim 3, comprising transforming a microorganism host cell with a vector according to any one of claims 10-12, culturing the transformed cells and obtaining from the cultured cells the said proteins, analogs, fragments and fusion polypeptides.

15. A method according to claim 14, wherein the host cell is *E. coli*, the vector is that according to claim 12 and the fusion polypeptide product is obtained from lysates of the culture of transformed host cells.

16. A diagnostic method for the detection of the presence of antibodies against *Schistosoma* in subjects, comprising contacting a test sample of said subjects' blood or other tissue fluids also containing subjects' antibodies with one or more of the proteins, analogs, fragments or fusion polypeptides according to any one of Claims 1-3, and determining binding of said proteins, analogs, fragments or fusion polypeptides to antibodies in said test sample.

FIG. 1

FIG. 2

FIG. 3

```
   1  AGATAGGTGCACAGTTTAAAATGCTGGTCCGATTGTCTGTTTTCTGAACGATGGATCTCCGCTACTTTGCTATTACAAAC    80
                                       M  D  L  R  Y  F  A  I  T  N

  81  GAGAAACATATTGTTTGGTATCGGTCGTTCACAAAACGTAGTTTTGAAGATGTAGTACTTAAATATCTGTTATGTAATCC   160
       E  K  H  I  V  W  Y  R  S  F  T  K  R  S  F  E  D  V  V  L  K  Y  L  L  C  N  P

 161  TCCAGATCAAGATATATATTATAGCGAAGGCGATTTCTCTCTTCACTGCTTAACCGTTTCAGGACTTTCCTTTATTGCGG   240
       P  D  Q  D  I  Y  Y  S  E  G  D  F  S  L  H  C  L  T  V  S  G  L  S  F  I  A  V

 241  TAACCGATCGAAACGCATCTCGACAAAAATTTCGAAATTTGGTTGTGGAGGTTTCTCGTAATTTTCTCAGTGATGGTACA   320
       T  D  R  N  A  S  R  Q  K  F  R  N  L  V  V  E  V  S  R  N  F  L  S  D  G  T

 321  AGATTTCAAAGGGCTAAAAGTGGTGGTAAAGATTGTTTACAACAGTCTTATAGTCTGAACTTGGAAGAGTTGATGGAATC   400
       R  F  Q  R  A  K  S  G  G  K  D  C  L  Q  Q  S  Y  S  L  N  L  E  E  L  M  E  S

 401  ACCCAACTCTTCTAAGGGAAATGGTTCGTTGGACCAGTTACGTAGTAATGTGAATACTGTAACAGCTGTAATGCAAGAGA   480
       P  N  S  S  K  G  N  G  S  L  D  Q  L  R  S  N  V  N  T  V  T  A  V  M  Q  E  N

 481  ATACTAGACTAGCGCTTCAACGAGGTGATCGGTTAAACAATTTGGTCAGTAAAACAGATGAACTTGCAGCCAGCGCTAAT   560
        T  R  L  A  L  Q  R  G  D  R  L  N  N  L  V  S  K  T  D  E  L  A  A  S  A  N

 561  CAATTCCAAACAACAGCCACAAAGGTGGCCCCGAAAAACTTGGTGGGAAAATTTCCGTATGAAATTAATTATTGTTGGTGT   640
       Q  F  Q  T  T  A  T  K  V  A  R  K  T  W  W  E  N  F  R  M  K  L  I  I  V  G  V

 641  TGTCGGTGGCATACTTCTAGTAATTGTGATAATTATACTCTGGCAGACTGGGGTGTTCAATAGCAAATCGCAACACCAGT   720
       V  G  G  I  L  L  V  I  V  I  I  L  W  Q  T  G  V  F  N  S  K  S  Q  H  Q  *

 721  AATAATAGCAACAGTAAATAATTAACTTCATATTGTTTACTACATGAAACAGAAAATGCAAAGGAACACCCATCTGAAAA   800
        *  *

 801  TTAGGTGAGATATTCGATAATGTGTTTTTTTTACTGGATCAATTTATACAACACGTCTTGTTAACATTTCAATTTTCTTT   880

 881  ATCGCGACACGTTCTCCTTTTGTTTCCCTCGTGACAGATAAATTTTACAAATGATGATGACAATGATTAATATTGCACAG   960

 961  TATATTGTAATTTTTATGCTATATTATAAATATACATACTTAAGTCTTTTTTTGTAATCACTATACCTACTACTAATCCT  1040

1041  GCATTAACTACTTCTCACTGATGAAATATCTTTTCTATATAGGCGCAGAGATTTAATGACAATTACATATCATATATATA  1120

1121  TATATATATATATATATATAGTGTTCGGAATGATTTTTTCTCTTAAACGAATTTATTTTTCACTCAACATCTATCT      1200

1201  ACTTTGTATGGGTGACTATCTTCCCAATGTATATCGATTATGTGTTCTTTATTGATCCCAATACACATTTTGTTTATTTT  1280

1281  CAATAACAATGACTTTTTGAAAGCCAAAGATTATCAATAAATGAAAGAAGATAAAAGAGGTACAAACTGTTTGATATCTT  1360

1361  TTTGTTGGAATTACACTTTGTAAACGCATGCATGTGTCTTTATGCGTGCGTGTGTGCATATATGTTCAAGTGGCTTCATA  1440

1441  ATAATACCACAACTATTCACTTTTATCATTATTTCCGTGCCCTGTCCTTTTTACTTCTCTACAGATAATTTAAAGTATAC  1520

1521  TCATTTCTGTACTATTTCTTCTTGCGGTTATTCTCATTACTATTAACATTATGAGAAAATGTGTATTTGCAATTTCTACT  1600

1601  CTGGAAGAATTCCTTCTTATTATTGATTAGTACTTATCATGGTTTTACTTTTTACCAGATTTAAATTAATATGTTTGCTC  1680

1681  AATATACATAAATTTTCTTTTCCTTTTTTTTAACAAAAATCATATTTGTCATGTATTGAATACCAACAAAATTGAATTC 1759
```

# FIG. 4

```
  1  M  D  L  R  Y  F  A  I  T  N   E  K  H  I  V  W  Y  R  S  F   T  K  R  S  F  E  D  V  V  L

 31  K  Y  L  L  C  N  P  P  D  Q   D  I  Y  Y  S  E  G  D  F  S   L  H  C  L  T  V  S  G  L  S

 61  F  I  A  V  T  D  R  N  A  S   R  Q  K  F  R  N  L  V  V  E   V  S  R  N  F  L  S  D  G  T

 91  R  F  Q  R  A  K  S  G  G  K   D  C  L  Q  Q  S  Y  S  L  N   L  E  E  L  M  E  S  P  N  S

121  S  K  G  N  G  S  L  D  Q  L   R  S  N  V  N  T  V  T  A  V   M  Q  E  N  T  R  L  A  L  Q

151  R  G  D  R  L  N  N  L  V  S   K  T  D  E  L  A  A  S  A  N   Q  F  Q  T  T  A  T  K  V  A

181  R  K  T  W  W  E  N  F  R  M   K  L  I  I  V  G  V  V  G  G   I  L  L  V  I  V  I  I  I  L

211  W  Q  T  G  V  F  N  S  K  S   Q  H  Q   223
```

# FIG. 5

FIG. 6

EP 0 554 064 A1

FIG. 7

FIG. 8

FIG. 9

FIG. 10

FIG. 11

## DOCUMENTS CONSIDERED TO BE RELEVANT

EP 93300573.8

| Category | Citation of document with indication, where appropriate, of relevant passages | Relevant to claim | CLASSIFICATION OF THE APPLICATION (Int. Cl.5) |
|---|---|---|---|
| A | WO - A - 91/09 621 (INSTITUT PASTEUR) * Abstract; claims * -- | 1,2,4, 5 | A 61 K 39/00 C 12 N 15/62 C 07 K 15/08 C 07 K 13/00 |
| A | US - A - 5 051 254 (STRAND) * Abstract; claims 2-7 * -- | 1-6 | C 07 H 21/04 A 61 K 39/395 C 12 N 15/63 G 01 N 33/566 |
| A | EP - A - 0 268 501 (TRANSGENE S.A.) * Abstract; claims * -- | 1-5,8- 14 | G 01 N 33/68 |
| A | EP - A - 0 318 879 (UNIVERSITY HOSPITALS OF CLEVELAND) * Abstract; claims * ---- | 4-6 | |

**TECHNICAL FIELDS SEARCHED (Int. Cl.5)**

A 61 K
C 12 N
C 07 H
C 07 K
G 01 N 33/00

The present search report has been drawn up for all claims

| Place of search | Date of completion of the search | Examiner |
|---|---|---|
| VIENNA | 24-03-1993 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P0401)